# EUROPEAN PATENT APPLICATION

(11) **EP 4 424 684 A1**
(43) Date of publication of application: **04.09.2024**
(21) Application number: 22886066.4
(22) Date of filing: 27.10.2022
(51) Int. Cl.: C07D 471/20, A61K 31/438, A61P 35/00

(54) **PYRROLOPYRAZOLE SPIRO COMPOUND**

(30) Priority: 27.10.2021 CN 202111257617; 19.11.2021 CN 202111402382; 14.01.2022 CN 202210045128; 18.10.2022 CN 202211275680
(71) Applicant: Chia Tai Tianqing Pharmaceutical Group Co., Ltd., Lianyungang, Jiangsu 222062 (CN); Medshine Discovery Inc., Nanjing, Jiangsu 210032 (CN)
(72) Inventor: FU, Xiangyu, Shanghai 200131 (CN); DING, Charles Z., Shanghai 200131 (CN); HU, Lihong, Shanghai 200131 (CN)
(74) Representative: Engelhard, Markus
(86) International application number: PCT/CN2022/127937
(87) International publication number: WO 2023/072191

(57) **Abstract**

**ABSTRACT**

Disclosed are a pyrrolopyrazole spiro compound, and the use thereof in preparing a drug for treating related diseases. The present invention specifically relates to a compound as represented by formula (I'), and a stereoisomer and a pharmaceutically acceptable salt thereof.

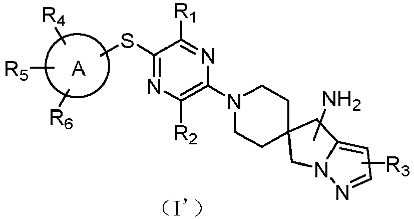

## Description

### CROSS-REFERENCE TO RELATED APPLICATIONS

The present application claims 1) the priority and benefit to the Chinese Patent Application No. 202111257617.8 filed with the China National Intellectual Property Administration on October 27, 2021, 2) the priority and benefit to the Chinese Patent Application No. 202111402382.7 filed with the China National Intellectual Property Administration on November 19, 2021, 3) the priority and benefit to the Chinese Patent Application No. 202210045128.4 filed with the China National Intellectual Property Administration on January 14, 2022, and 4) the priority and benefit to the Chinese Patent Application No. 202211275680.9 filed with the China National Intellectual Property Administration on October 18, 2022, which are incorporated herein by reference in their entireties.

### TECHNICAL FIELD

The present application relates to a pyrrolopyrazole spiro compound and use thereof for preparing a medicament for treating a related disease, and in particular to a compound of formula (I), a stereoisomer thereof, and a pharmaceutically acceptable salt thereof.

### BACKGROUND

SHP2 is a member of the protein tyrosine phosphatase (PTP) family and is a non-receptor type phosphatase that catalyzes the protein tyrosine dephosphorylation reaction. SHP2 is encoded by PTP non-receptor 11 (PTPN11) and is widely expressed in the human body, playing an important role in downstream signaling pathways of growth factor receptors. As an upstream protein of many important pathways including RAS-ERK, PI3K-AKT, JAK-STAT, and the like, SHP2 regulates a variety of physiological functions such as cell proliferation, differentiation, migration and apoptosis. SHP2 has also been found to be involved in the PD-1/PD-L1 pathway, playing a role in immune regulation. Studies have shown that SHP2 is indispensable for RAS-driven tumors. SHP2 is upstream of RAS, mediating the activation of the RAS-ERK pathway. This function is primarily achieved by dephosphorylation of RAS-GAP. When stimulated by various growth factors, SHP2 dephosphorylates tyrosine phosphorylation sites of RTKs, and prevents p120RasGAP from inhibiting Ras activation by dephosphorylating the binding sites of p120RasGAP to RTKs, thereby serving the purpose of activating the RAS pathway.

From the N-terminus, the SHP2 protein contains two Src homology domains, N-SH2 and C-SH2, followed by a catalytically active PTP domain. Normal SHP2 is in a self-inhibiting state, with the active catalytic site being blocked by N-SH2. When Tyr542 and Tyr580 are phosphorylated, a conformational change occurs and SHP2 is activated. When SHP2 undergoes a mutation (e.g., E76K, etc.), the conformation of N-SH2 is changed greatly, failing to block the active catalytic site, and thus SHP2 is activated without the need for phosphorylation. SHP2 mutations have been found in a variety of tumors.

Since the PTP domain plays a role of catalyzing phosphate, its catalytic site is highly polar, which makes the development of small-molecule drugs that directly act on the catalytic site challenging. Allosteric inhibitors are another approach to inhibiting SHP2. The SHP2 allosteric inhibitors act as a "glue", maintaining SHP2 in its self-inhibiting state. At present, a plurality of SHP2 allosteric inhibitors have entered the clinical research phase and have shown excellent efficacy in diseases such as NSCLC.

In summary, allosteric inhibitors targeting SHP2 are capable of becoming an advanced cancer treatment regime.

### SUMMARY

The present application provides a compound of formula (I'), a stereoisomer thereof, or a pharmaceutically acceptable salt thereof: 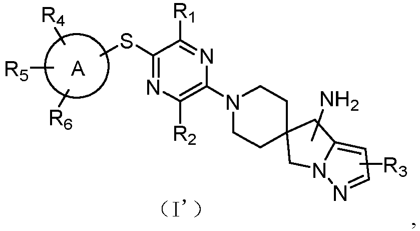 wherein
ring A is selected from the group consisting of aryl and heteroaryl;
R₁ is selected from the group consisting of H, deuterium, NH₂, and C₁₋₆ alkyl optionally substituted with one or more halogens, CN, or OH;
R₂ is selected from the group consisting of deuterium, H, halogen, OH, CN, COOH, -C(=O)-Cₗ₋₆ alkyl, -COO-C₁₋₆ alkyl, C₁₋₆ alkyl, and -C(=O)NH₂, wherein the -C(=O)-Cₗ₋₆ alkyl, -COO-C₁₋₆ alkyl, C₁₋₆ alkyl and -C(=O)NH₂ are each independently optionally substituted with 1, 2, or 3 Rₐ;
R₃ is selected from the group consisting of H, halogen, OH, NOz, CN, C₁₋₆ alkyl, C₁₋₆ alkoxy, and C₁₋₆ alkylamino, wherein the C₁₋₆ alkyl, C₁₋₆ alkoxy and C₁₋₆ alkylamino are each independently optionally substituted with 1, 2, or 3 R_{b};
R₄, R₅, and R₆ are each independently selected from the group consisting of H, deuterium, halogen, NH₂, NOz, CN, OH, C₁₋₆ alkyl, C₁₋₆ alkoxy, and -NH-O-C₁₋₆ alkyl, wherein the NH₂, C₁₋₆ alkyl, C₁₋₆ alkoxy and -NH-O-C₁₋₆ alkyl are each independently optionally substituted with 1, 2, or 3 R_{c};
Rₐ, R_{b}, and R_{c} are each independently selected from the group consisting of deuterium, halogen, OH, NH₂, CN, and C₁₋₃ alkyl.

The present application provides a compound of formula (I), a stereoisomer thereof, or a pharmaceutically acceptable salt thereof: 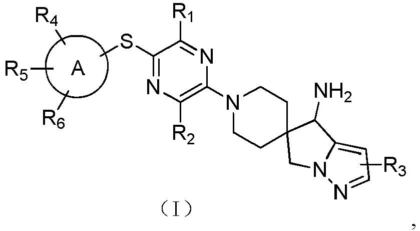 wherein
ring A is selected from the group consisting of aryl and heteroaryl;
R₁ is selected from the group consisting of H, deuterium, NH₂, and C₁₋₆ alkyl optionally substituted with one or more halogens, CN, or OH;
R₂ is selected from the group consisting of deuterium, H, halogen, OH, CN, COOH, -C(=O)-Cₗ₋₆ alkyl, -COO-C₁₋₆ alkyl, C₁₋₆ alkyl, and -C(=O)NH₂, wherein the -C(=O)-Cₗ₋₆ alkyl, -COO-C₁₋₆ alkyl, C₁₋₆ alkyl and -C(=O)NH₂ are each independently optionally substituted with 1, 2, or 3 Rₐ;
R₃ is selected from the group consisting of H, halogen, OH, NOz, CN, C₁₋₆ alkyl, C₁₋₆ alkoxy, and C₁₋₆ alkylamino, wherein the C₁₋₆ alkyl, C₁₋₆ alkoxy and C₁₋₆ alkylamino are each independently optionally substituted with 1, 2, or 3 R_{b};
R₄, R₅, and R₆ are each independently selected from the group consisting of H, deuterium, halogen, NH₂, NOz, CN, OH, C₁₋₆ alkyl, C₁₋₆ alkoxy, and -NH-O-C₁₋₆ alkyl, wherein the NH₂, C₁₋₆ alkyl, C₁₋₆ alkoxy and -NH-O-C₁₋₆ alkyl are each independently optionally substituted with 1, 2, or 3 R_{c};
Rₐ, R_{b}, and R_{c} are each independently selected from the group consisting of deuterium, halogen, OH, NH₂, CN, and C₁₋₃ alkyl.

In some embodiments, ring A is selected from the group consisting of aryl and 5- to 6-membered heteroaryl, wherein the 5- to 6-membered heteroaryl comprises 1, 2, or 3 heteroatoms or heteroatom groups each independently selected from the group consisting of N, O, S, and NH.

In some embodiments, R₁ is selected from the group consisting of H, deuterium, NH₂, and C₁₋₃ alkyl optionally substituted with one or more halogens, CN, or OH. In some embodiments, R₁ is selected from the group consisting of H, deuterium, NH₂, and C₁₋₃ alkyl optionally substituted with one or more halogens. In some embodiments, R₁ is selected from the group consisting of H, deuterium, NH₂, and C₁₋₃ alkyl optionally substituted with one or more fluorine. In some embodiments, R₁ is selected from the group consisting of H, deuterium, NH₂, CH₃, CHF₂, CH₂F, and CF₃.

In some embodiments, R₂ is selected from the group consisting of deuterium, F, Cl, Br, I, CN, COOH, -C(=O)-C₁₋₃ alkyl, -COO-C₁₋₃ alkyl, C₁₋₃ alkyl, and -C(=O)NH₂, wherein the -C(=O)-C₁₋₃ alkyl, -COO-C₁₋₃ alkyl, C₁₋₃ alkyl and - C(=O)NH₂ are each independently optionally substituted with 1, 2, or 3 Rₐ.

In some embodiments, R₃ is selected from the group consisting of H, F, Cl, Br, I, NO₂, CN, C₁₋₃ alkyl, C₁₋₃ alkoxy, and C₁₋₃ alkylamino, wherein the C₁₋₃ alkyl, C₁₋₃ alkoxy and C₁₋₃ alkylamino are each independently optionally substituted with 1, 2, or 3 R_{b}.

In some embodiments, R₄, R₅, and R₆ are each independently selected from the group consisting of H, deuterium, F, Cl, Br, I, NH₂, NO₂, CN, OH, C₁₋₃ alkyl, C₁₋₃ alkoxy, and -NH-O-C₁₋₃ alkyl, wherein the NH₂, C₁₋₃ alkyl, C₁₋₃ alkoxy and -NH-O-C₁₋₃ alkyl are each independently optionally substituted with 1, 2, or 3 R_{c}.

The present application provides a compound of formula (I), a stereoisomer thereof, or a pharmaceutically acceptable salt thereof: 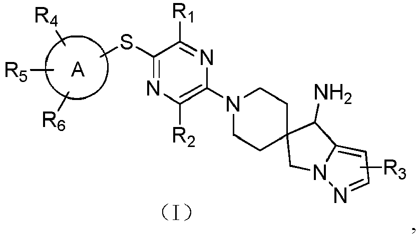 wherein
ring A is selected from the group consisting of aryl and 5- to 6-membered heteroaryl, wherein the 5- to 6-membered heteroaryl comprises 1, 2, or 3 heteroatoms or heteroatom groups each independently selected from the group consisting of N, O, S, and NH;
R₁ is selected from the group consisting of H, deuterium, NH₂, CH₃, CHF₂, CH₂F, and CF₃;
R₂ is selected from the group consisting of deuterium, F, Cl, Br, I, CN, COOH, -C(=O)-C₁₋₃ alkyl, -COO-C₁₋₃ alkyl, C₁₋₃ alkyl, and -C(=O)NH₂, wherein the -C(=O)-C₁₋₃ alkyl, -COO-C₁₋₃ alkyl, C₁₋₃ alkyl and -C(=O)NH₂ are each independently optionally substituted with 1, 2, or 3 Rₐ;
R₃ is selected from the group consisting of H, F, Cl, Br, I, NO₂, CN, C₁₋₃ alkyl, C₁₋₃ alkoxy, and C₁₋₃ alkylamino, wherein the C₁₋₃ alkyl, C₁₋₃ alkoxy and C₁₋₃ alkylamino are each independently optionally substituted with 1, 2, or 3 R_{b};
R₄, R₅, and R₆ are each independently selected from the group consisting of H, deuterium, F, Cl, Br, I, NH₂, NO₂, CN, OH, C₁₋₃ alkyl, C₁₋₃ alkoxy, and -NH-O-C₁₋₃ alkyl, wherein the NH₂, C₁₋₃ alkyl, C₁₋₃ alkoxy and -NH-O-C₁₋₃ alkyl are each independently optionally substituted with 1, 2, or 3 R_{c};
each Rₐ is independently selected from the group consisting of deuterium, F, Cl, Br, I, OH, and NH₂;
each R_{b} is independently selected from the group consisting of deuterium, F, Cl, Br, I, and OH;
each R_{c} is independently selected from the group consisting of deuterium, F, Cl, Br, I, NH₂, and C₁₋₃ alkyl.

In some embodiments, Rₐ, R_{b}, and R_{c} are each independently selected from the group consisting of halogen, OH, NH₂, and C₁₋₃ alkyl. In some embodiments, Rₐ, R_{b}, and R_{c} are each independently selected from the group consisting of halogen, OH, and C₁₋₃ alkyl. In some embodiments, Rₐ, R_{b}, and R_{c} are each independently selected from the group consisting of fluorine, OH, and C₁₋₃ alkyl. In some embodiments, Rₐ, R_{b}, and Rₑ are each independently selected from the group consisting of fluorine, OH, and CH₃.

In some embodiments, each Rₐ is independently selected from the group consisting of deuterium, F, Cl, Br, I, OH, and NH₂;
each R_{b} is independently selected from the group consisting of deuterium, F, Cl, Br, I, and OH;
each R_{c} is independently selected from the group consisting of deuterium, F, Cl, Br, I, NH₂, and C₁₋₃ alkyl.

In some embodiments, each Rₐ is independently selected from the group consisting of F, Cl, OH, and NH₂. In some embodiments, each Rₐ is independently selected from OH.

In some embodiments, each R_{b} is independently selected from the group consisting of F, Cl, and OH.

In some embodiments, each R_{c} is independently selected from the group consisting of F, Cl, Br, I, and C₁₋₃ alkyl. In some embodiments, each R_{c} is independently selected from the group consisting of F, Cl, Br, I, NH₂, and CH₃. In some embodiments, each R_{c} is independently selected from the group consisting of F, Cl, NH₂, and CH₃.

In some embodiments, each R_{c} is independently selected from the group consisting of F and C₁₋₃ alkyl.

In some embodiments of the present application, each R_{c} described above is independently selected from the group consisting of F and CH₃, while the other variables are as defined herein.

In some embodiments of the present application, R₁ described above is selected from the group consisting of H, CH₃, and CHF₂, while the other variables are as defined herein.

In some embodiments of the present application, R₁ described above is selected from the group consisting of CH₃ and CHF₂, while the other variables are as defined herein.

In some embodiments of the present application, R₂ described above is selected from the group consisting of deuterium, F, Cl, Br, I, CH₃, and -C(=O)NH₂, wherein the CH₃ and -C(=O)NH₂ are each independently optionally substituted with 1, 2, or 3 Rₐ, while the other variables are as defined herein.

In some embodiments of the present application, R₂ described above is selected from the group consisting of Cl, - CH₂OH, -CH₃, and -C(=O)NH₂, while the other variables are as defined herein.

In some embodiments of the present application, R₂ described above is selected from the group consisting of Cl, - CH₂OH, and -C(=O)NH₂, while the other variables are as defined herein.

In some embodiments of the present application, R₃ described above is selected from the group consisting of H, F, Cl, and CH₃, while the other variables are as defined herein.

In some embodiments of the present application, R₃ described above is selected from H, while the other variables are as defined herein.

In some embodiments of the present application, R₄, R₅, and R₆ described above are each independently selected from the group consisting of H, deuterium, F, Cl, Br, I, NH₂, NO₂, CN, OH, =O, CH₃, -OCH₃, and -NH-O-CH₃, wherein the NH₂, CH₃, -OCH₃ and -NH-O-CH₃ are each independently optionally substituted with 1, 2, or 3 R_{c}, while the other variables are as defined herein.

In some embodiments of the present application, R₄, R₅, and R₆ described above are each independently selected from the group consisting of F, Cl, NH₂, -NH-O-CH₃, -NH-CH₃, CH₃, CF₃, and CHF₂, while the other variables are as defined herein.

In some embodiments of the present application, the structural unit 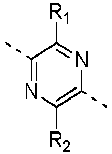 described above is selected from the group consisting of 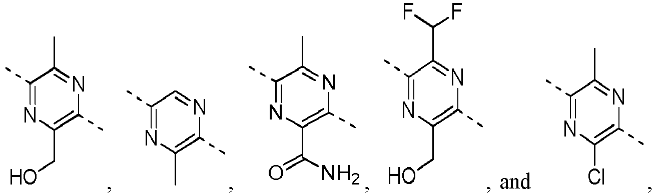 while the other variables are as defined herein.

In some embodiments of the present application, the structural unit 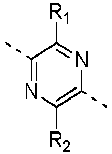 described above is selected from the group consisting of 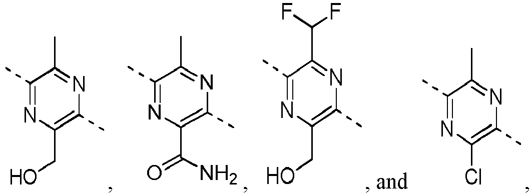 while the other variables are as defined herein.

In some embodiments of the present application, the structural unit 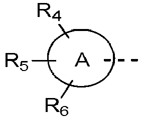 described above is selected from the group consisting of 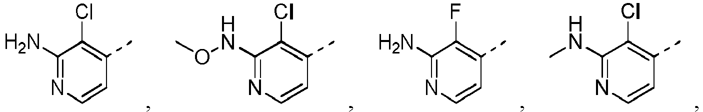 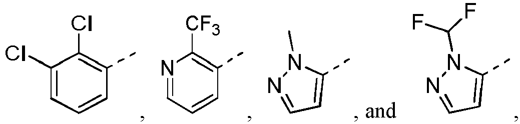 while the other variables are as defined herein.

In some embodiments of the present application, ring A described above is selected from the group consisting of C₆₋₁₀ aryl and 5- to 10-membered heteroaryl. In some embodiments of the present application, ring A described above is selected from the group consisting of C₆₋₁₀ aryl and 5- to 6-membered heteroaryl. In some embodiments of the present application, ring A described above is selected from the group consisting of phenyl and 5- to 6-membered heteroaryl. In some embodiments of the present application, ring A described above is selected from the group consisting of phenyl, pyrazolyl, and pyridinyl, while the other variables are as defined herein.

In some aspects of the present application:
ring A described above is selected from the group consisting of phenyl, pyrazolyl, and pyridinyl; or ring A described above is selected from pyridinyl;
R₂ described above is selected from the group consisting of deuterium, F, Cl, Br, I, CH₃, and -C(=O)NH₂, wherein the CH₃ and -C(=O)NH₂ are each independently optionally substituted with 1, 2, or 3 Rₐ; or R₂ described above is selected from the group consisting of Cl, -CH₂OH, CH₃, and -C(=O)NH₂; or R₂ described above is selected from the group consisting of Cl, -CH₂OH, and -C(=O)NH₂; or R₂ described above is selected from -CH₂OH;
R₃ described above is selected from the group consisting of H, F, Cl, and CH₃; or R₃ described above is selected from H;
R₄, R₅, and R₆ described above are each independently selected from the group consisting of F, Cl, NH₂, -NH-O-CH₃, -NH-CH₃, CH₃, CF₃, and CHF₂,
while the other variables are as defined herein.

Some other embodiments of the present application are derived from any combination of the variables described above.

The present application provides a compound of formula (I), a stereoisomer thereof, or a pharmaceutically acceptable salt thereof, wherein the compound of formula (I) is selected from the group consisting of compounds of formula (I)-A and formula (I)-B:

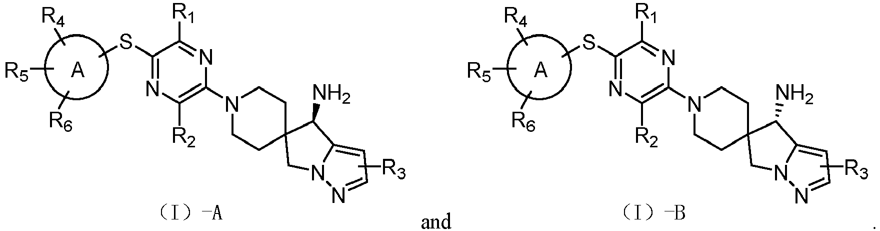

In some embodiments of the present application, the compound described above is selected from the group consisting of 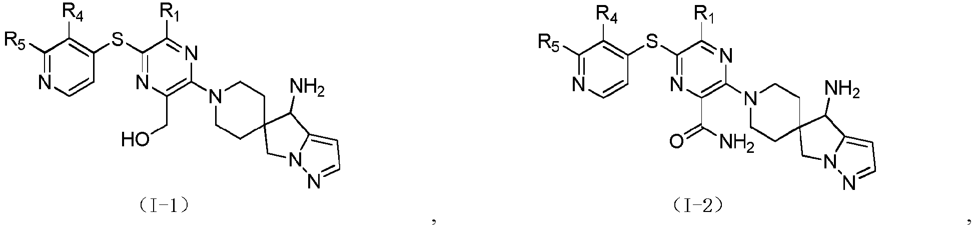 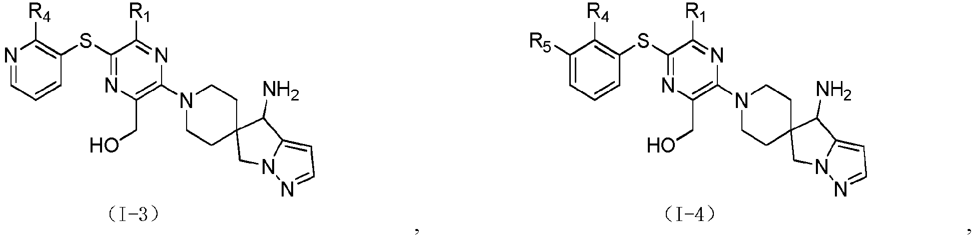 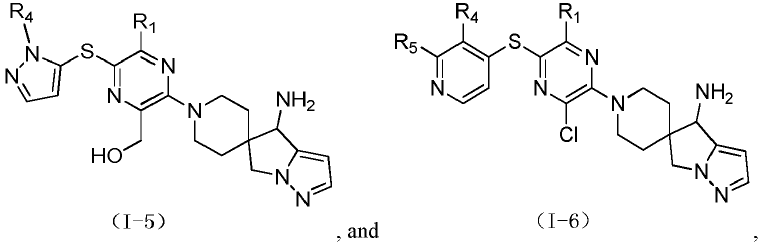 wherein R₁, Rₐ, and R₅ are as defined herein.

In some embodiments of the present application, the compound described above is selected from the group consisting of 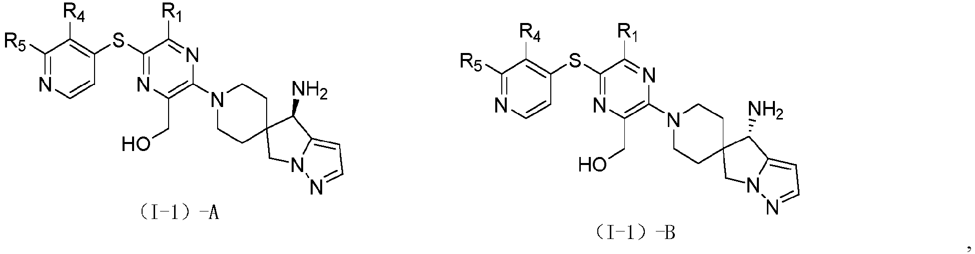 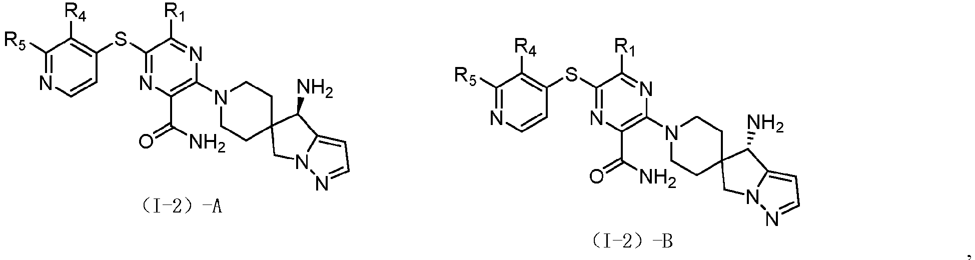 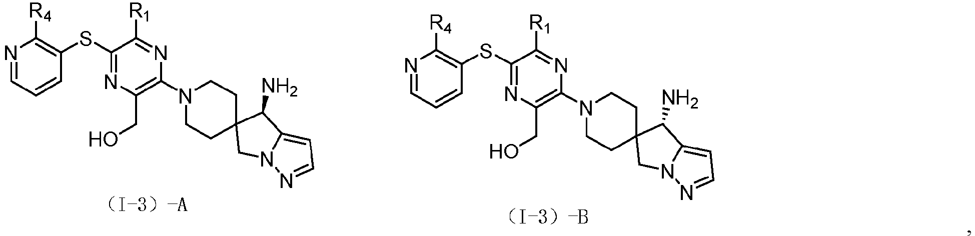 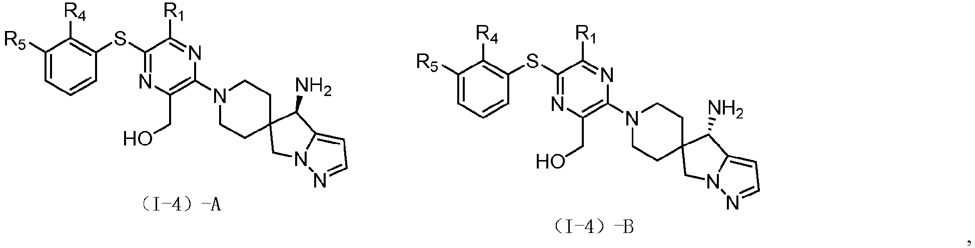 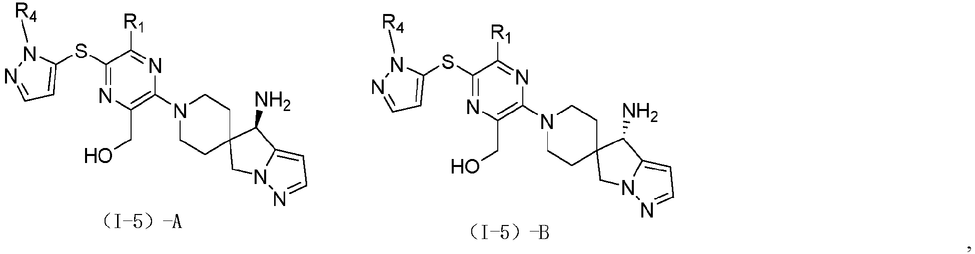 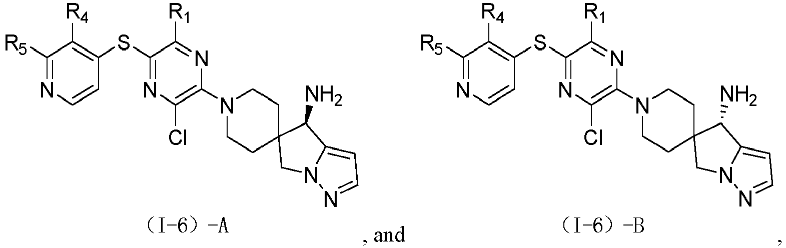 wherein R₁, Rₐ, and R₅ are as defined herein.

The present application further provides a compound, a stereoisomer thereof, or a pharmaceutically acceptable salt thereof, wherein the compound is selected from the group consisting of

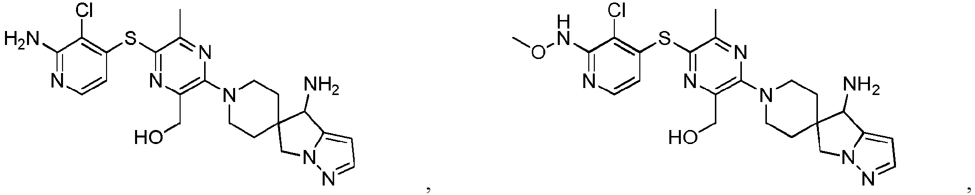

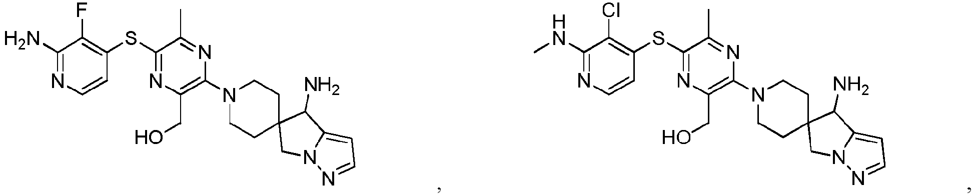

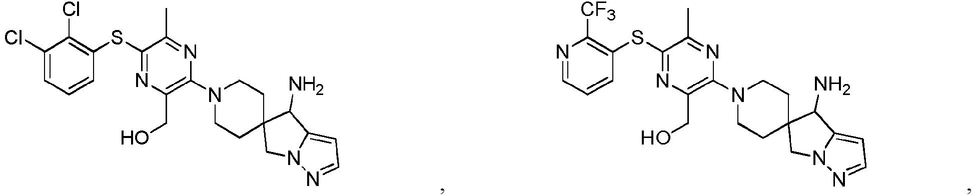

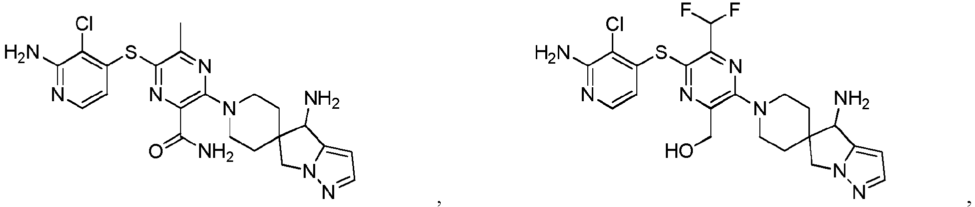

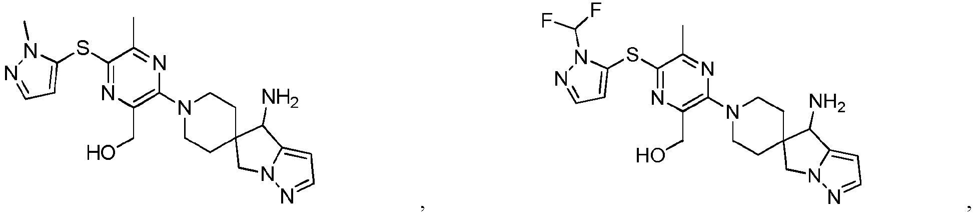

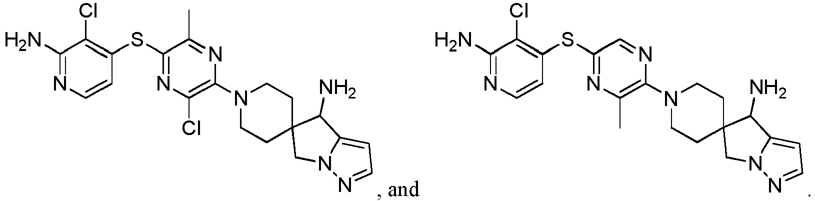

In some embodiments of the present application, the compound described above is selected from the group consisting of

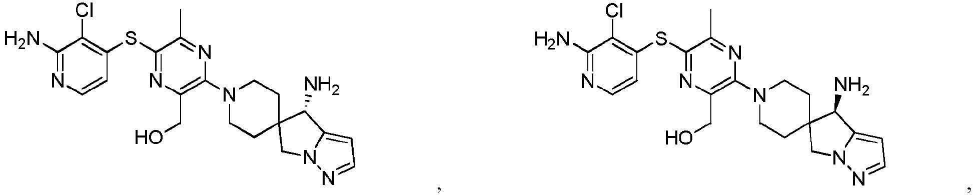

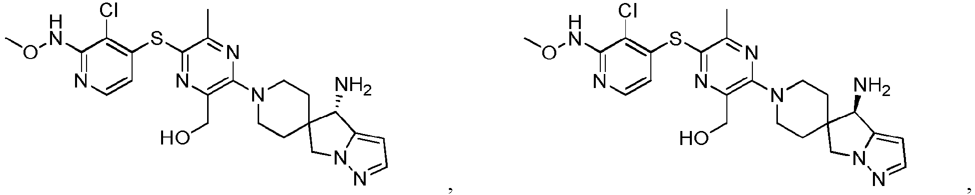

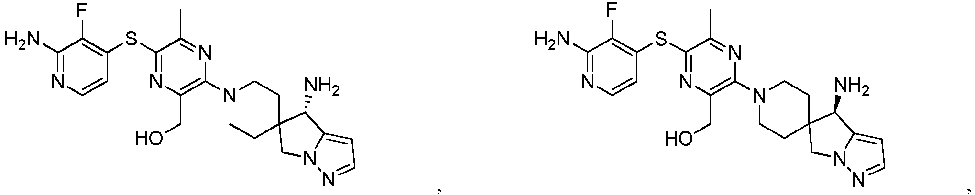

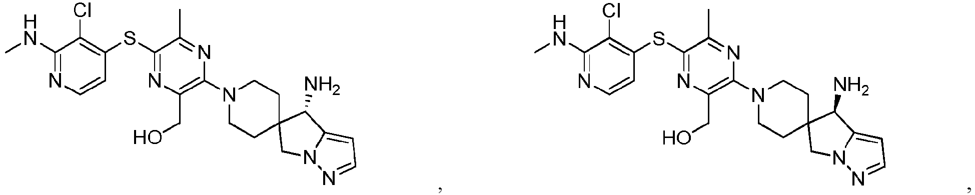

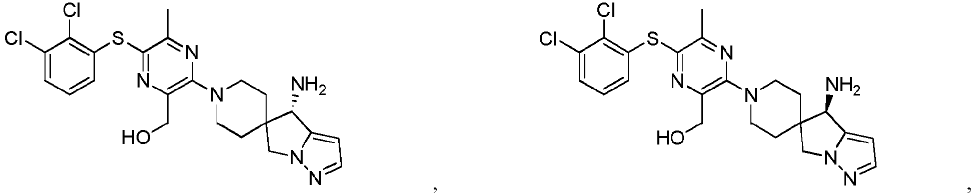

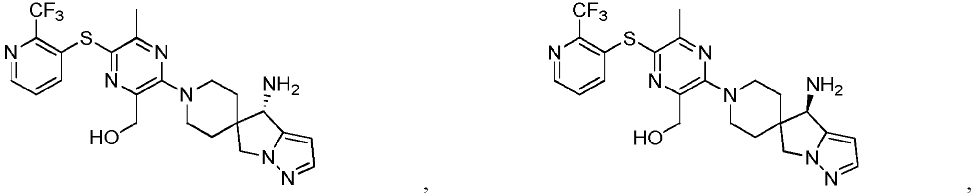

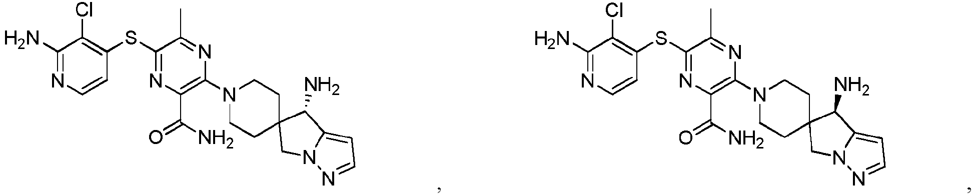

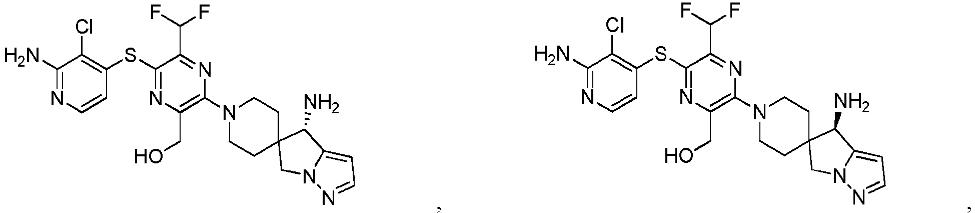

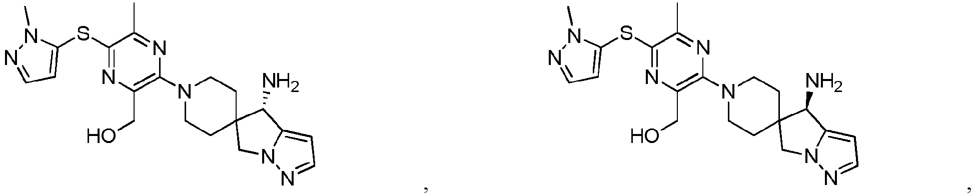

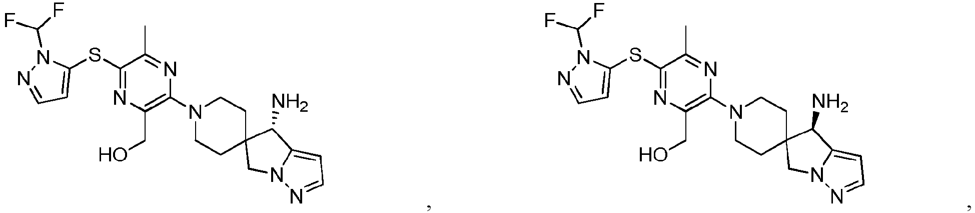

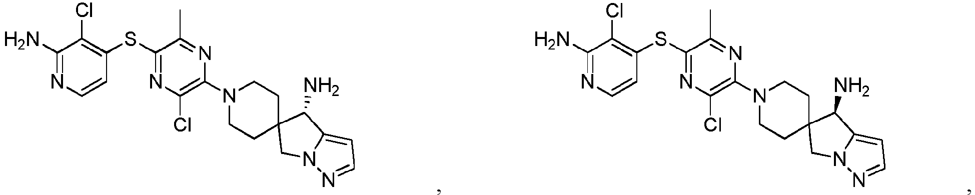

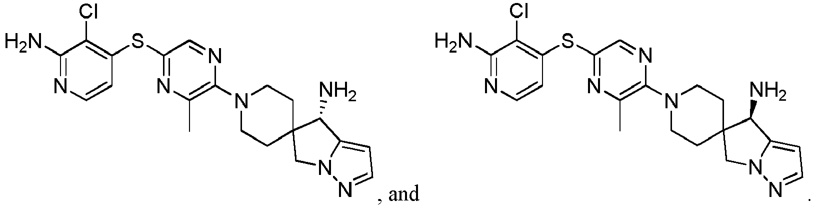

In another aspect, the present application relates to a pharmaceutical composition, comprising the compound, the stereoisomer thereof, or the pharmaceutically acceptable salt thereof disclosed herein. In some embodiments, the pharmaceutical composition disclosed herein further comprises a pharmaceutically acceptable excipient.

In another aspect, the present application relates to a method for preventing or treating an SHP2 protein-related disease in a mammal, comprising administering to a mammal, preferably a human, in need of the treatment a therapeutically effective amount of the compound, the stereoisomer thereof, or the pharmaceutically acceptable salt thereof, or the pharmaceutical composition thereof disclosed herein.

In another aspect, the present application relates to use of the compound, the stereoisomer thereof, or the pharmaceutically acceptable salt thereof, or the pharmaceutical composition thereof disclosed herein for preparing a medicament for preventing or treating an SHP2 protein-related disease.

In another aspect, the present application relates to use of the compound, the stereoisomer thereof, or the pharmaceutically acceptable salt thereof, or the pharmaceutical composition thereof disclosed herein for preventing or treating an SHP2 protein-related disease.

In another aspect, the present application relates to the compound, the stereoisomer thereof, or the pharmaceutically acceptable salt thereof, or the pharmaceutical composition thereof disclosed herein for use in preventing or treating an SHP2 protein-related disease.

In some embodiments, the SHP2 protein-related disease is selected from cancer; or the SHP2 protein-related disease is selected from the group consisting of lung cancer and pancreatic cancer.

### Technical Effects

The present application provides a novel SHP2 allosteric inhibitor, which has high *in-vivo* and *in-vitro* inhibitory activity on SHP2 and can be used as a novel and more effective regime for treating cancer. Specifically, it can inhibit the activity of kinases or tumor cells (MIAPACA2_PANCREAS and NCIH358_LUNG) *in vitro* and has excellent pharmacokinetic and pharmacodynamic properties *in vivo.*

### Definitions and Explanations

Unless otherwise stated, the following terms and phrases used herein are intended to have the following meanings. A particular term or phrase, unless otherwise specifically defined, should not be considered indefinite or unclear, but construed according to its common meaning. When referring to a trade name, it is intended to refer to its corresponding commercial product or its active ingredient.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound disclosed herein, which is prepared from the compound having particular substituents discovered by the present application and a relatively nontoxic acid or base. When the compound disclosed herein contains a relatively acidic functional group, a base addition salt can be obtained by allowing such a compound to be in contact with a sufficient amount of a base in a pure solution or a suitable inert solvent. Pharmaceutically acceptable base addition salts include sodium, potassium, calcium, ammonium, organic amine or magnesium salts, or similar salts. When the compound disclosed herein contains a relatively basic functional group, an acid addition salt can be obtained by allowing such a compound to be in contact with a sufficient amount of an acid in a pure solution or a suitable inert solvent. Certain specific compounds disclosed herein contain both basic and acidic functional groups that allow the compounds to be converted into either base or acid addition salt.

The pharmaceutically acceptable salts disclosed herein can be synthesized from a parent compound having an acidic or basic group using conventional chemical methods. In general, such salts are prepared by subjecting the compounds in a free acid or base form to a reaction with a stoichiometric amount of appropriate base or acid in water or an organic solvent or a mixture thereof.

The compounds disclosed herein can be in the form of a geometric isomer or stereoisomer. All such compounds are contemplated herein, including *cis-* and *trans*-isomers, (-)- and (+)-enantiomers, (*R*)- and (*S*)-enantiomers, diastereoisomers, (*D*)-isomers, (*L*)-isomers, and racemic mixtures and other mixtures thereof, such as an enantiomer or diastereoisomer enriched mixture, all of which are encompassed within the scope of the present application. The substituents such as alkyl may have an additional asymmetric carbon atom. All these isomers and mixtures thereof are encompassed within the scope of the present application.

Unless otherwise stated, the term "enantiomer" or "optical isomer" refers to stereoisomers that are mirror images of each other.

Unless otherwise stated, the term "*cis-trans* isomer" or "geometric isomer" results from the inability of a single bond of a ring carbon atom or a double bond to rotate freely.

Unless otherwise stated, the term "diastereoisomer" refers to stereoisomers whose molecules have two or more chiral centers and are not mirror images of each other.

Unless otherwise stated, "(+)" stands for dextrorotation, "(-)" stands for levorotation, and "(±)" stands for racemization.

Unless otherwise stated, the absolute configuration of a stereogenic center is represented by a wedged solid bond ( 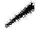 ) and a wedged dashed bond ( 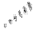 ), and the relative configuration of a stereogenic center is represented by a straight solid bond ( 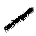 ) and a straight dashed bond ( 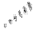 ). A wavy line ( 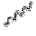 ) represents a wedged solid bond ( 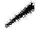 ) or a wedged dashed bond ( 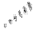 ), or a wavy line ( 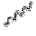 ) represents a straight solid bond ( 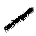 ) and a straight dashed bond ( 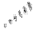 ).

Unless otherwise stated, the term "tautomer" or "tautomeric form" means that different functional isomers are in dynamic equilibrium at room temperature and can be rapidly converted into each other. If tautomers are possible (e.g., in a solution), the chemical equilibrium of the tautomers can be achieved. For example, a proton tautomer, also known as a prototropic tautomer, includes the interconversion by proton transfer, such as keto-enol isomerization and imine-enamine isomerization. A valence isomer includes the interconversion by recombination of some bonding electrons. A specific example of the keto-enol tautomerization is the interconversion between tautomers pentane-2,4-dione and 4-hydroxypent-3-en-2-one.

Unless otherwise stated, the term "enriched in one isomer", "isomer enriched", "enriched in one enantiomer", or "enantiomer enriched" means that the content of one of the isomers or enantiomers is less than 100% and more than or equal to 60%, 70%, 80%, 90%, 95%, 96%, 97%, 98%, 99%, 99.5%, 99.6%, 99.7%, 99.8% or 99.9%.

Unless otherwise stated, the term "isomeric excess" or "enantiomeric excess" refers to the difference between the relative percentages of two isomers or enantiomers. For example, if the content of one of the isomers or enantiomers is 90% and the content of the other isomer or enantiomer is 10%, the isomeric or enantiomeric excess (ee) is 80%.

Optically active (*R*)- and (*S*)-isomers and *D* and *L* isomers can be prepared by chiral synthesis or chiral reagents or other conventional techniques. An enantiomer of a certain compound disclosed herein can be prepared by asymmetric synthesis or derivatization using a chiral additive, wherein the resulting diastereoisomeric mixture is separated and the auxiliary group is cleaved so as to provide the desired pure enantiomer. Alternatively, when the molecule contains a basic functional group (such as amino) or an acidic functional group (such as carboxyl), the compound reacts with an appropriate optically active acid or base to form a salt of the diastereoisomer, which is then subjected to diastereoisomeric resolution through conventional methods in the art to give the pure enantiomer. Furthermore, the enantiomer and the diastereoisomer are generally isolated through chromatography using a chiral stationary phase, optionally in combination with chemical derivatization (e.g., carbamate generated from amines).

The compound disclosed herein may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen can be substituted with deuterium to form a deuterated drug, and the bond formed by deuterium and carbon is firmer than that formed by common hydrogen and carbon. Compared with an un-deuterated drug, the deuterated drug has the advantages of reduced toxic side effects, increased stability, enhanced efficacy, prolonged biological half-life, and the like. All isotopic variations of the compound disclosed herein, whether radioactive or not, are encompassed within the scope of the present application. The term "optional" or "optionally" means that the subsequently described event or circumstance may, but not necessarily, occur, and the description includes instances where the event or circumstance occurs and instances where it does not.

The term "substituted" means that one or more hydrogen atoms on a specific atom are substituted with substituents, wherein the substituents may include deuterium and hydrogen variants, as long as the valence of the specific atom is normal and the substituted compound is stable. When the substituent is oxygen (i.e., =O), it means that two hydrogen atoms are substituted. Substitution with oxygen does not occur on aromatic groups. The term "optionally substituted" means that an atom can be or cannot be substituted with a substituent. Unless otherwise specified, the type and number of the substituent may be arbitrary as long as being chemically achievable.

When any variable (e.g., R) occurs once or more in the constitution or structure of a compound, the definition of the variable in each case is independent. Thus, for example, if a group is substituted with 0-2 R, the group can be optionally substituted with up to two R, and the definition of R in each case is independent. Furthermore, a combination of the substituent and/or the variant thereof is permissible only if the combination can result in a stable compound.

When the number of a connecting group is 0, for example, -(CRR)₀-, it means that the connecting group is a single bond.

When one of the variables is selected from a single bond, it means that the two groups linked by the single bond are connected directly. For example, in A-L-Z, when L represents a single bond, it means that the structure is actually A-Z.

When a substituent is absent, it means that the substituent does not exist. For example, when X is absent in A-X, the structure of A-X is actually A. When it is not specified by which atom the listed substituent is linked to the group to be substituted, the substituent can be linked via any atom of the group. For example, pyridinyl as a substituent can be linked to the group to be substituted through any carbon atom on the pyridine ring. When the direction for connection of the listed connecting group is not specified, the direction for connection is arbitrary. For example, when the connecting group L contained in 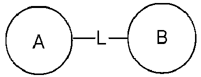 is -M-W-, -M-W- can either connect ring A and ring B in a direction same as left-to-right reading order to form 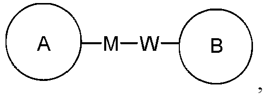 or connect ring A and ring B in a direction opposite to the left-to-right reading order to form 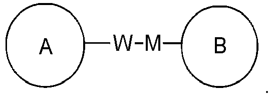 A combination of the connecting group, the substituent, and/or the variant thereof is permissible only if the combination can result in a stable compound.

Unless otherwise specified, when a group has one or more connectable sites, any one or more of the sites of the group may be connected to other groups by chemical bonds. If there is no designated connecting mode for chemical bonds and H atoms are present at a connectable site, when the connectable site is connected to chemical bonds, the number of the H atoms at the connectable site is correspondingly reduced based on the number of the connected chemical bonds, so that the resulting groups have corresponding valences. The chemical bond that connects the site and another group may be represented by a straight solid bond ( 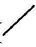 ), a straight dashed bond ( 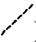 ), or a wavy line (). For example, the straight solid bond in -OCH₃ refers to being connected to another group via the oxygen atom in the group; the straight dashed bond in 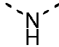 refers to being connected to another group via two ends of the nitrogen atom in the group; the wavy line in 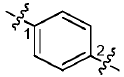 refers to being connected to another group via the carbon atoms at positions 1 and 2 in the phenyl group; 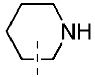 means that any connectable site on the piperidinyl can be connected to another group via 1 chemical bond in at least 4 connecting modes: 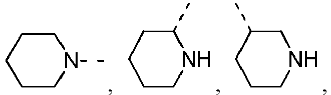 and 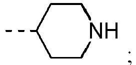 even if -N- is connected with an H atom, 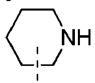 includes the connection mode of 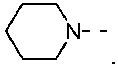 but when 1 chemical bond is connected to a site the number of H at that site is correspondingly reduced by 1 and a monovalent piperidinyl is thus formed.

Unless otherwise specified, the number of atoms on a ring is generally defined as the member number of the ring. For example, "5- to 7-membered ring" refers to a "ring" on which 5 to 7 atoms are arranged in a circle.

The term "alkyl" refers to a linear or branched saturated hydrocarbon group consisting of carbon atoms. Unless otherwise specified, the term "C₁₋₆ alkyl" refers to a linear or branched saturated hydrocarbon group consisting of 1 to 6 carbon atoms; the term "C₁₋₃ alkyl" refers to a linear or branched saturated hydrocarbon group consisting of 1 to 3 carbon atoms. The C₁₋₃ alkyl includes C₁₋₂, C₂₋₃ alkyl, and the like, and may be monovalent (e.g., methyl), divalent (e.g., methylene), or polyvalent (e.g., methenyl). Examples of C₁₋₃ alkyl include, but are not limited to, methyl (Me), ethyl (Et), propyl (including n-propyl and isopropyl), and the like.

The term "alkoxyl" refers to -O-alkyl. Unless otherwise specified, the term "C₁₋₆ alkoxy" refers to those alkyl groups that each contain 1 to 6 carbon atoms and are linked to the rest part of the molecule via an oxygen atom; the term "C₁₋₃ alkoxy" refers to those alkyl groups that each contain 1 to 3 carbon atoms and are linked to the rest part of the molecule via an oxygen atom. The C₁₋₃ alkoxy includes C₁₋₂, C₂₋₃, C₃ and C₂ alkoxy, and the like. Examples of C₁₋₃ alkoxy include, but are not limited to, methoxy, ethoxy, propoxy (including n-propoxy and isopropoxy), and the like. The term "C₁₋₆ alkylamino" refers to -NH-alkyl. Unless otherwise specified, the term "C₁₋₆ alkylamino" refers to those alkyl groups that each contain 1 to 6 carbon atoms and are linked to the rest part of the molecule via an amino group; the term "C₁₋₃ alkylamino" refers to those alkyl groups that each contain 1 to 3 carbon atoms and are linked to the rest part of the molecule via an amino group. The C₁₋₃ alkylamino includes C₁₋₂, C₃ and C₂ alkylamino, and the like. Examples of C₁₋₃ alkylamino include, but are not limited to, -NHCH₃, -N(CH₃)₂, -NHCH₂CH₃, - N(CH₃)CH₂CH₃, -NHCH₂CH₂CH₃, -NHCH₂(CH₃)₂, and the like.

Unless otherwise specified, the term "halo" or "halogen", by itself or as part of another substituent, refers to a fluorine, chlorine, bromine, or iodine atom.

The term "aryl" refers to an all-carbon aromatic monocyclic or fused polycyclic group having a conjugated π-electron system. For example, aryl may have 6-20 carbon atoms, 6-14 carbon atoms, or 6-12 carbon atoms. Non-limiting examples of aryl include, but are not limited to, phenyl, naphthyl, anthryl, 1,2,3,4-tetrahydronaphthalene, and the like.

The term "heteroaryl" refers to a monocyclic or fused polycyclic ring system that comprises at least one ring atom selected from the group consisting of N, O, and S, such as 1, 2, 3, or 4 ring atoms selected from the group consisting of N, O, and S, with the remaining ring atoms being C, and that has at least one aromatic ring. Preferably, heteroaryl has a single 5- to 8-membered or 5- to 6-membered ring, or has a plurality of fused rings comprising 6 to 14 ring atoms, in particular 6 to 10 ring atoms.

Unless otherwise specified, the terms "5- to 6-membered heteroaromatic ring" and "5- to 6-membered heteroaryl" are used interchangeably herein, and the term "5- to 6-membered heteroaryl" refers to a monocyclic group consisting of 5 to 6 ring atoms with a conjugated π-electron system, of which 1, 2, 3 or 4 ring atoms are heteroatoms independently selected from the group consisting of O, S and N, and the rest are carbon atoms. The nitrogen atom is optionally quaternized, and the nitrogen and sulfur heteroatoms can be optionally oxidized (i.e., NO and S(O)ₚ, wherein p is 1 or 2). The 5- to 6-membered heteroaryl can be connected to the rest of the molecule through a heteroatom or a carbon atom. The 5- to 6-membered heteroaryl includes 5-membered heteroaryl and 6-membered heteroaryl. Examples of the 5- to 6-membered heteroaryl include, but are not limited to, pyrrolyl (including N-pyrrolyl, 2-pyrrolyl, 3-pyrrolyl, and the like), pyrazolyl (including 2-pyrazolyl, 3-pyrazolyl, and the like), imidazolyl (including *N*-imidazolyl, 2-imidazolyl, 4-imidazolyl, 5-imidazolyl, and the like), oxazolyl (including 2-oxazolyl, 4-oxazolyl, 5-oxazolyl, and the like), triazolyl (including 1*H*-1,2,3-triazolyl, 2*H*-1,2,3-triazolyl, 1*H*-1,2,4-triazolyl, 4*H-*1,2,4-triazolyl, and the like), tetrazolyl, isoxazolyl (including 3-isoxazolyl, 4-isoxazolyl, 5-isoxazolyl, and the like), thiazolyl (including 2-thiazolyl, 4-thiazolyl, 5-thiazolyl, and the like), furanyl (including 2-furanyl, 3-furanyl, and the like), thienyl (including 2-thienyl, 3-thienyl, and the like), pyridinyl (including 2-pyridinyl, 3-pyridinyl, 4-pyridinyl, and the like), pyrazinyl, or pyrimidinyl (including 2-pyrimidinyl, 4-pyrimidinyl, and the like).

Unless otherwise specified, Cₙ₋ₙ₊ₘ or Cₙ-Cₙ₊ₘ includes any one of the specific cases of n to n+m carbon atoms. For example, C₁₋₁₂ includes C₁, C₂, C₃, C₄, C₅, C₆, C₇, C₈, C₉, C₁₀, C₁₁ and C₁₂. Also, any range within n to n+m may be included. For example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc. Similarly, n- to n+m-membered represents that the number of atoms on the ring is n to n+m. For example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring. n- to n+m-membered also represents any range within n to n+m. For example, 3- to 12-membered ring includes 3- to 6-membered ring, 3- to 9-membered ring, 5- to 6-membered ring, 5- to 7-membered ring, 6- to 7-membered ring, 6- to 8-membered ring, 6- to 10-membered ring, and the like.

The term "treat", "treating", or "treatment" means administering a compound or formulation described herein to ameliorate or eliminate a disease or one or more symptoms associated with the disease, including:
(i) inhibiting a disease or disease state, i.e., arresting its progression; and
(ii) alleviating a disease or disease state, i.e., causing its regression.

The term "prevent", "preventing", or "prevention" means administering a compound or formulation described herein to prevent a disease or one or more symptoms associated with the disease, including preventing the occurrence of the disease or disease state in a mammal, particularly when such a mammal is predisposed to the disease state but has not yet been diagnosed with it.

The term "therapeutically effective amount" refers to an amount of the compound disclosed herein for (i) treating or preventing a specific disease, condition, or disorder; (ii) alleviating, ameliorating, or eliminating one or more symptoms of a specific disease, condition, or disorder, or (iii) preventing or delaying onset of one or more symptoms of a specific disease, condition, or disorder described herein. The amount of the compound disclosed herein composing the "therapeutically effective amount" varies depending on the compound, the disease state and its severity, the administration regimen, and the age of the mammal to be treated, but can be determined routinely by those skilled in the art in accordance with their knowledge and the present disclosure.

The term "pharmaceutical composition" refers to a mixture consisting of one or more of the compounds or the salts thereof disclosed herein and a pharmaceutically acceptable excipient. The pharmaceutical composition is intended to facilitate the administration of the compound disclosed herein to an organism.

The term "pharmaceutically acceptable excipient" refers to those that do not have a significant irritating effect on an organism and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, or water.

The word "comprise" and variations thereof such as "comprises" or "comprising" will be understood in an open, non-exclusive sense, i.e., "including but not limited to".

Therapeutic dosages of the compound of the present application may be determined by, for example, the specific use of a treatment, the route of administration of the compound, the health and condition of a patient, and the judgment of a prescribing physician. The proportion or concentration of the compound of the present application in a pharmaceutical composition may not be constant and depends on a variety of factors including dosages, chemical properties (e.g., hydrophobicity), and routes of administration. For example, the compound of the present application may be provided for parenteral administration by a physiological buffered aqueous solution containing about 0.1-10%w/v of the compound. Certain typical dosages range from about 1 µg/kg body weight/day to about 1 g/kg body weight/day. In certain embodiments, the dosage ranges from about 0.01 mg/kg body weight/day to about 100 mg/kg body weight/day. The dosage is likely to depend on such variables as the type and degree of progression of the disease or disorder, the general health condition of a particular patient, the relative biological potency of the compound selected, the excipient formulation, and its route of administration. Effective doses can be extrapolated from dose-response curves derived from *in vitro* or animal model test systems.

The chemical reactions of the specific embodiments of the present application are conducted in a proper solvent that must be suitable for the chemical changes in the present application and the reagents and materials required. In order to obtain the compounds disclosed herein, it is sometimes necessary for those skilled in the art to modify or select a synthesis procedure or a reaction process based on the existing embodiments.

The compounds disclosed herein can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

In some embodiments, the compound disclosed herein may be prepared by those skilled in the art through the following general route and using methods known in the art:

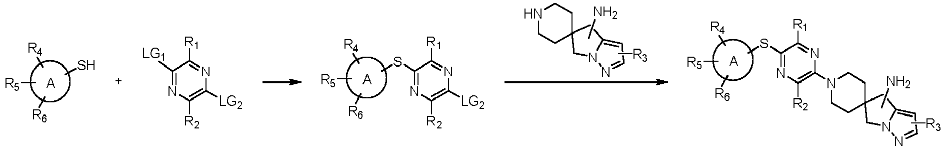

LG1 and LG2 are leaving groups, such as halogen and -O-O₂SR (R = optionally substituted C₁₋₆ alkyl).

The compound disclosed herein may be structurally confirmed by conventional methods well known to those skilled in the art; if the present application relates to the absolute configuration of the compound, this absolute configuration may be confirmed by means of conventional techniques in the art. For example, in single crystal X-ray diffraction (SXRD), intensity data of diffraction of the single crystal grown are collected with a Bruker D8 venture diffractometer, with the light source being Cu-Kα radiation and the scanning mode being ϕ/ω scanning; after related data are collected, a direct method (Shelxs97) is further employed to analyze the crystal structure, and thus the absolute configuration can be confirmed.

The solvents used herein are commercially available.

The following abbreviations are used herein: aq for water; HATU for O-(7-azabenzotriazol-1-yl)-*N*,*N*,*N*,*N-*tetramethyluronium hexafluorophosphate; EDC for *N*-(3-dimethylaminopropyl)-*N'*-ethylcarbodiimide hydrochloride; *m*-CPBA for 3-chloroperoxybenzoic acid; eq for equivalent; CDI for carbonyldiimidazole; DCM for dichloromethane; PE for petroleum ether; DIAD for diisopropyl azodicarboxylate; DMF for *N,N-*dimethylformamide; DMSO for dimethyl sulfoxide; EtOAc for ethyl acetate; EtOH for ethanol; MeOH for methanol; CBz for benzyloxycarbonyl, an amine protecting group; BOC for t-butoxycarbonyl, an amine protecting group; HOAc for acetic acid; NaCNBH₃ for sodium cyanoborohydride; r.t. for room temperature; O/N for overnight; THF for tetrahydrofuran; BoczO for di-*tert*-butyl dicarbonate; TFA for trifluoroacetic acid; DIPEA for diisopropylethylamine; SOCl₂ for thionyl chloride; CS₂ for carbon disulphide; TsOH for *p*-toluenesulfonic acid; NFSI for *N-*fluoro-*N-*(phenylsulfonyl)benzenesulfonamide; NCS for 1-chloropyrrolidine-2,5-dione; *n*-Bu₄NF for tetrabutylammonium fluoride; iPrOH for 2-propanol; mp for melting point; and LDA for lithium diisopropylamide.

### DETAILED DESCRIPTION

The present application is described in detail below by way of examples. However, this is by no means disadvantageously limiting the scope of the present application. The compounds disclosed herein can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application. It will be apparent to those skilled in the art that various changes and modifications can be made to the specific embodiments without departing from the spirit and scope of the present application.

### Example 1: Compound 1

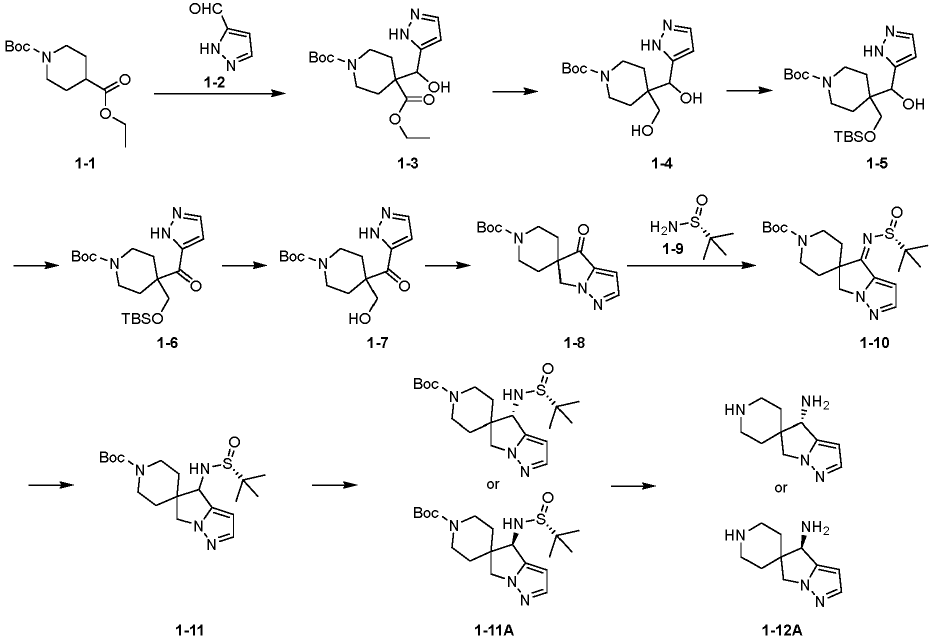

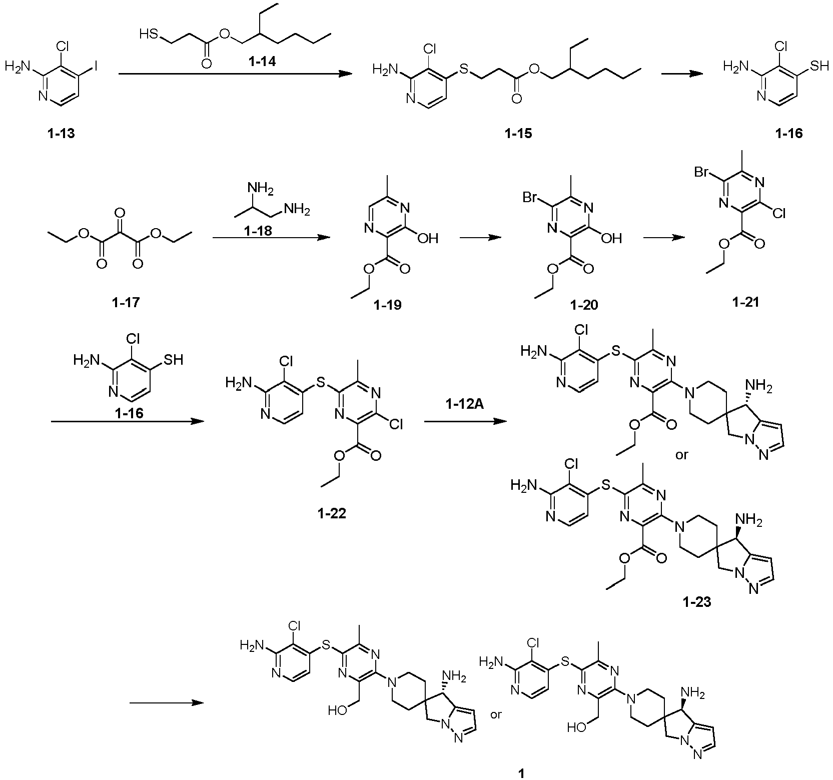

Step A: Under nitrogen atmosphere, a solution of compound **1-1** (8.9 g, 34.59 mmol, 1 eq) in tetrahydrofuran (100 mL) was cooled to -70, and then lithium diisopropylamide (2 mol/L, 20.75 mL, 1.2 eq) was added. After the mixture was stirred at -70 °C for 1 h, compound **1-2** (3.66 g, 38.05 mmol, 1.1 eq) was added. After the mixture was further stirred at 25 °C for 12 h, ethyl acetate (200 mL) was added. The system was then washed with brine (300 mL × 3), dried over anhydrous sodium sulfate, filtered, and concentrated under reduced pressure to give a crude product, which was then purified by preparative high performance liquid chromatography (column: Phenomenex luna C18 (250 mm × 70 mm, 10 µm), mobile phase: [water (0.225% formic acid)-acetonitrile]; acetonitrile%: 25%-55%, 22 min) to give compound **1-3.** MS (ESI) m/z: 254.2, 298.2 [M+H⁺-100, M+H⁺-56].

Step B: Lithium aluminum hydride (376.95 mg, 9.93 mmol, 1.3 eq) was added to a solution of compound **1-3** (2.7 g, 7.64 mmol, 1 eq) in tetrahydrofuran (30 mL) at 0 °C. After the mixture was stirred at 25 °C for 12 h, a saturated sodium sulfate solution was added to quench the reaction until no bubbles were generated. Ethyl acetate (100 mL) was then added, and the mixture was filtered. The filtrate was washed with brine (100 mL × 2), dried over anhydrous sodium sulfate, filtered, and concentrated under reduced pressure to give compound **1-4.** MS (ESI) m/z: 256.1 [M+H⁺-56].

Step C: Imidazole (491.93 mg, 7.23 mmol, 1.5 eq) and *tert*-butyldimethylsilyl chloride (871.29 mg, 5.78 mmol, 708.36 µL, 1.2 eq) were added to a solution of compound **1-4** (1.5 g, 4.82 mmol, 1 eq) in *N*,*N*-dimethylformamide (20 mL). After the mixture was stirred at 25 °C for 1 h, ethyl acetate (80 mL) was added. The system was then washed with brine (80 mL × 5), dried over anhydrous sodium sulfate, filtered, and concentrated under reduced pressure to give compound **1-5.** MS (ESI) m/z: 426.3 [M+H⁺].

Step D: Dess-Martin reagent (2.63 g, 6.20 mmol, 1.2 eq) was added to a solution of compound **1-5** (2.2 g, 5.17 mmol, 1 eq) in dichloromethane (20 mL), and the mixture was stirred at 25 °C for 12 h. After the reaction was completed, a sodium sulfite solution (40 mL) and a saturated sodium bicarbonate solution (40 mL) were added, and the mixture was stirred at 25 °C for 30 min and extracted with dichloromethane (50 mL × 2). The organic phases were combined, washed with brine, dried over anhydrous sodium sulfate, filtered, and concentrated under reduced pressure to give a crude product, which was then purified by silica gel column chromatography (eluent: percentage of ethyl acetate in petroleum ether = 0%-50%) to give compound **1-6.** MS (ESI) m/z: 324.5 [M+H⁺-100].

Step E: Tetrabutylammonium fluoride (1 mol/L, 2.66 mL, 1.5 eq) was added to a solution of compound **1-6** (750 mg, 1.77 mmol, 1 eq) in tetrahydrofuran (20 mL), and the mixture was stirred at 25 °C for 12 h. After the reaction was completed, ethyl acetate (50 mL) was added. The system was then washed with brine (50 mL × 5), dried over anhydrous sodium sulfate, filtered, and concentrated under reduced pressure to give compound **1-7.** MS (ESI) m/z: 210.0 [M+H⁺-100].

Step F: Triphenylphosphine (578.22 mg, 2.20 mmol, 1.1 eq) and diisopropyl azodicarboxylate (445.78 mg, 2.20 mmol, 428.63 µL, 1.1 eq) were added to a solution of compound **1-7** (620 mg, 2.00 mmol, 1 eq) in tetrahydrofuran (10 mL), and the mixture was stirred at 25 °C for 12 h and concentrated under reduced pressure to give a crude product, which was then purified by silica gel column chromatography (eluent: percentage of ethyl acetate in petroleum ether = 0%-20%) to give compound **1-8.** MS (ESI) m/z: 236.2 [M+H⁺-56].

Step G: Tetraethoxytitanium (156.59 mg, 686.47 µmol, 142.35 µL, 2 eq) was added to a solution of compounds **1-8** (100 mg, 343.24 µmol, 1 eq) and **1-9** (124.80 mg, 1.03 mmol, 3 eq) in toluene (5 mL), and the mixture was stirred at 110 °C under nitrogen atmosphere for 12 h. After the reaction was completed, compound **1-10** was obtained, which was used directly in the solution in the next step. MS (ESI) m/z: 395.5 [M+H⁺].

Step H: Sodium borohydride (130 mg, 3.44 mmol, 10.01 eq) was added to a solution of compound **1-10** (135.42 mg, 343.24 µmol, 1 eq) in toluene (3 mL) under nitrogen atmosphere at -70 °C, and the mixture was stirred at -70 °C for 1 h. Methanol (5 mL) was added to quench the reaction, and then 20 mL of ethyl acetate was added. The mixture was filtered, and the filtrate was washed with brine (20 mL × 3), dried over anhydrous sodium sulfate, filtered, and concentrated under reduced pressure to give a crude product, which was then purified by a preparative high performance liquid chromatography column (column model: Phenomenex luna C18 (150 mm × 25 mm × 10 µm), mobile phase: [0.225% aqueous formic acid solution-acetonitrile]; gradient: 30%-60%) to give compound 1-11. MS (ESI) m/z: 397.5 [M+H⁺]. Compound **1-11** was separated by preparative SFC (column model: DAICEL CHIRALPAK IC (250 mm × 30 mm × 10 µm), mobile phases: supercritical carbon dioxide as phase A and methanol (0.1% ammonium hydroxide) as phase B, gradient (B%): 35%-35%) to give compound **1-11A.** MS (ESI) m/z: 397.5 [M+H⁺]. Compound **1-11A** was tested by SFC (column model: Chiralpak IG-3 (50 mm × 4.6 mm × 3 µm), mobile phases: supercritical carbon dioxide as phase A and methanol (0.05% diethylamine) as phase B, gradient (B%): 5%-40%), and the following results were obtained: compound **1-11A** had a retention time of 1.294 min and an e.e. value of 100.00%.

Step I: Hydrogen chloride in ethyl acetate (4 mol/L, 1 mL, 9.33 eq) was added to a solution of compound **1-11A** (170 mg, 428.70 µmol, 1 eq) in ethyl acetate (1 mL), and the mixture was stirred at 20 °C for 30 min. After the reaction was completed, ethyl acetate (5 mL) was added. The mixture was filtered, and the filter cake was dried under reduced pressure to give compound **1-12A.**

Step J: Compound **1-13** (10 g, 39.30 mmol, 1 eq) and compound **1-14** (10.30 g, 47.16 mmol, 1.2 eq) were dissolved in dioxane (100 mL), and *N*,*N*-diisopropylethylamine (10.16 g, 78.60 mmol, 13.69 mL, 2 eq), 4,5-bis(diphenylphosphino)-9,9-dimethylxanthene (2.27 g, 3.93 mmol, 0.1 eq), and tris(dibenzylideneacetone)dipalladium (1.80 g, 1.96 mmol, 0.05 eq) were added. The reaction mixture was purged three times with nitrogen atmosphere, and then under nitrogen atmosphere, the reaction system was heated to 105 °C and stirred for 12 h. After the reaction was completed, the reaction solution was cooled to 25 °C and filtered. The filtrate was concentrated to give a crude product, and then n-heptane (50 mL) and ethyl acetate (5 mL) were added. The mixture was stirred at 25 °C for 1 h and filtered, and the filter cake was dried in vacuum to give compound **1-15.** MS (ESI) m/z: 345.2 [M+H⁺].

Step K: Compound **1-15** (13.2 g, 38.27 mmol, 1 eq) was dissolved in tetrahydrofuran (130 mL), and a solution of sodium methoxide in methanol (5.4 mol/L, 9.21 mL, 1.3 eq) was added. The reaction mixture was stirred at 25 °C for 1 h. After the reaction was completed, the reaction solution was directly concentrated to give a crude product, which was then dissolved in water (50 mL) and washed with ethyl acetate (50 mL × 2). The aqueous phase was adjusted to pH 6 with an aqueous hydrochloric acid solution (1 mol/L) and filtered. The filter cake was dried in vacuum to give compound **1-16.** MS (ESI) m/z: 161.1 [M+H⁺].

Step L: Compound **1-18** (29.79 g, 401.95 mmol, 34.33 mL, 1 eq) was dissolved in ethanol (350 mL), and **1-17** (70 g, 401.95 mmol, 61.95 mL, 1 eq) was added at 0 °C. The reaction mixture was stirred at 25 °C for 2 h. The reaction solution was heated to 80 °C and stirred for 20 h. After the reaction was completed, the reaction solution was directly concentrated to give a crude product, which was then subjected to column chromatography (silica gel, ethyl acetate:methanol = 1:0 to 1:1) to give compound **1-19.** MS (ESI) m/z: 183.1 [M+H⁺].

Step M: Compound **1-19** (51.46 g, 282.47 mmol, 1 eq) was dissolved in *N*,*N*-dimethylformamide (500 mL), and *N-*bromosuccinimide (52.79 g, 296.60 mmol, 1.05 eq) was added at 0 °C. The reaction mixture was stirred at 25 °C for 1 h. After the reaction was completed, water (500 mL) and ethyl acetate (700 mL) were added to the reaction solution, and the reaction mixture was stirred for 2 min. After the reaction system was separated into layers, the aqueous phase was extracted with ethyl acetate (700 mL). The organic phases were combined, washed with 10% brine (500 mL × 5), dried over anhydrous sodium sulfate, and filtered. The filtrate was concentrated to give a crude product, which was then subjected to column chromatography (silica gel, petroleum ether:ethyl acetate = 1:0 to 7:1) to give compound **1-20.** MS (ESI) m/z: 261.0, 263.0 [M+H⁺].

Step N: Triphenylphosphine (18.08 g, 68.95 mmol, 3 eq) was dissolved in dioxane (200 mL), and *N-*chlorosuccinimide (9.36 g, 70.10 mmol, 3.05 eq) was added. The reaction mixture was stirred at 25 °C for 0.5 h, and compound **1-20** (6 g, 22.98 mmol, 1 eq) was added. The reaction solution was heated to 100 °C and stirred for 1 h. After the reaction was completed, the reaction mixture was cooled to 25 °C, and the reaction solution was poured into water (600 mL). The mixture was extracted with methyl *tert*-butyl ether (400 mL × 2). The organic phases were combined, washed with saturated brine (400 mL), dried over anhydrous sodium sulfate, and filtered. The filtrate was concentrated to give a crude product, which was then subjected to column chromatography (silica gel, petroleum ether:ethyl acetate = 10:1 to 5:1) to give compound **1-21.** MS (ESI) m/z: 279.0 [M+H⁺].

Step O: Compound **1-21** (5.6 g, 20.03 mmol, 1 eq) and compound **1-16** (3.22 g, 20.03 mmol, 1 eq) were dissolved in dioxane (80 mL), and *N,N*-diisopropylethylamine (7.7 g, 60.10 mmol, 10.47 mL, 3 eq), 4,5-bis(diphenylphosphino)-9,9-dimethylxanthene (1.16 g, 2.00 mmol, 0.1 eq), and tris(dibenzylideneacetone)dipalladium (917.30 mg, 1.00 mmol, 0.05 eq) were added. The reaction mixture was purged three times with nitrogen atmosphere, and then under nitrogen atmosphere, the reaction system was heated to 100 °C and stirred for 5 h. After the reaction was completed, the reaction solution was cooled to 25 °C and filtered. The filtrate was concentrated to give a crude product, and then ethyl acetate (50 mL) was added. The mixture was stirred at 25 °C for 1 h and filtered, and the filter cake was dried under reduced pressure to give compound **1-22.** MS (ESI) m/z: 359.1 [M+H⁺].

Step P: Compound **1-22** (1.90 g, 5.29 mmol, 1 eq) and compound **1-12A** (1.82 g, 6.88 mmol, 1.3 eq, dihydrochloride) were dissolved in *N*-methylpyrrolidinone (20 mL), and potassium carbonate (3.65 g, 26.45 mmol, 5 eq) was added. The reaction mixture was heated to 80 °C and stirred for 1 h. After the reaction was completed, the reaction solution was cooled to 25 °C, and water (100 mL) was added. The mixture was filtered, and the filter cake was dried under reduced pressure to give compound **1-23.** MS (ESI) m/z: 515.3 [M+H⁺].

Step E: Compound **1-23** (1.1 g, 2.14 mmol, 1 eq) was dissolved in tetrahydrofuran (15 mL), and diisobutylaluminum hydride (1 mol/L, 8.54 mL, 4 eq) was added at 0 °C under nitrogen atmosphere. The reaction mixture was stirred at 25 °C for 1 h, and a saturated sodium sulfate solution was added to quench the reaction until no bubbles emerged. The mixture was then stirred at 25 °C for 30 min and filtered. The filtrate was concentrated under reduced pressure to give a crude product, which was then separated and purified by preparative high performance liquid chromatography (column model: Waters Xbridge BEH C18 (250 mm × 50 mm × 10 µm); mobile phase: [0.05% ammonium hydroxide-acetonitrile]; gradient: 15%-45%) to give compound 1. MS (ESI) m/z: 473.3 [M+H⁺]. ¹H NMR (400 MHz, DMSO-d6) δ = 7.65 (d, J = 5.6 Hz, 1H), 7.40 (d, J = 1.2 Hz, 1H), 6.34 (s, 2H), 6.05 (d, J = 1.6 Hz, 1H), 5.75 (d, J = 5.2 Hz, 1H), 5.45 (t, J = 5.6 Hz, 1H), 4.49 (d, J = 5.6 Hz, 2H), 4.15 (d, J = 10.8 Hz, 1H), 4.00 - 3.80 (m, 4H), 3.25 - 3.19 (m, 2H), 2.41 (s, 3H), 1.97 - 1.92 (m, 3H), 1.80 - 1.74 (m, 1H), 1.68 - 1.64 (m, 1H), 1.54-1.51 (m, 1H).

### Example 2: Compound 2

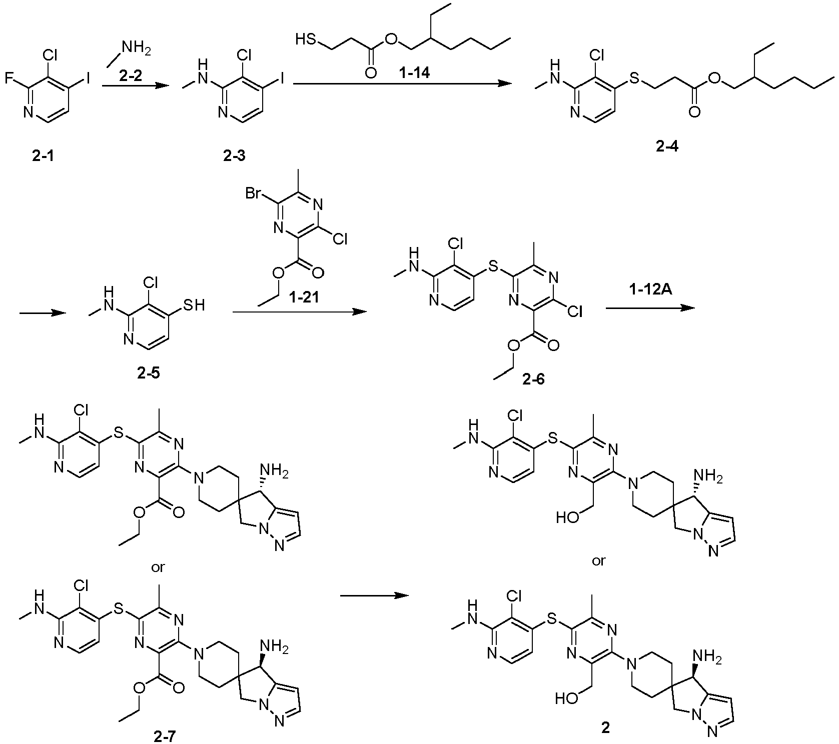

Step A: A solution of **2-2** in tetrahydrofuran (2 mol/L, 10 mL, 5.15 eq) was added to a solution of compound **2-1** (1 g, 3.88 mmol, 1 eq) in dimethyl sulfoxide (10 mL) under nitrogen atmosphere, and the mixture was stirred at 70 °C for 0.5 h. After the reaction was stopped, water (100 mL) was added, and the mixture was filtered to give compound **2-3.** MS (ESI) m/z: 268.9 [M+H⁺].

Step B: Compound **2-3** (0.89 g, 3.31 mmol, 1 eq) and compound **1-14** (868.60 mg, 3.98 mmol, 1.2 eq) were dissolved in dioxane (10 mL), and *N*,*N*-diisopropylethylamine (856.86 mg, 6.63 mmol, 1.15 mL, 2 eq), 4,5-bis(diphenylphosphino)-9,9-dimethylxanthene (95.90 mg, 165.5 µmol, 0.05 eq), and palladium acetate (37.21 mg, 165.75 µmol, 0.05 eq) were added. The reaction mixture was purged three times with nitrogen atmosphere, and then under nitrogen atmosphere, the reaction system was heated to 100 °C and stirred for 1 h. After the reaction was completed, the reaction solution was cooled to 15 °C. Ethyl acetate (30 mL) was then added, and the mixture was filtered. The filtrate was concentrated to give a crude product, and then petroleum ether (20 mL) was added. The mixture was filtered, and the filtrate was concentrated and subjected to column chromatography (silica gel, petroleum ether:ethyl acetate = 1:0 to 20:1) to give compound **2-4.** MS (ESI) m/z: 359.1 [M+H⁺].

Step C: Compound **2-4** (1.19 g, 3.30 mmol, 1 eq) was dissolved in tetrahydrofuran (10 mL), and a solution of sodium methoxide in methanol (5.4 mol/L, 1.22 mL, 2 eq) was added. The reaction mixture was stirred at 15 °C for 0.5 h. After the reaction was completed, the reaction solution was directly concentrated to give a crude product, which was then dissolved in water (20 mL). The system was then adjusted to pH 6 with 1 mol/L hydrochloric acid and washed with ethyl acetate (20 mL × 2). After the aqueous phase was concentrated, ethanol (20 mL) was added for dissolution, and the mixture was filtered and concentrated under reduced pressure to give compound **2-5.** MS (ESI) m/z: 175.1 [M+H⁺].

Step D: Compound **2-5** (0.386 g, 2.21 mmol, 1.1 eq) and compound **1-21** (561.61 mg, 2.01 mmol, 1 eq) were dissolved in dioxane (5 mL), and *N*,*N*-diisopropylethylamine (779.03 mg, 6.03 mmol, 1.05 mL, 3 eq), 4,5-bis(diphenylphosphino)-9,9-dimethylxanthene (116.26 mg, 200.92 µmol, 0.1 eq), and tris(dibenzylideneacetone)dipalladium (91.99 mg, 100.46 µmol, 0.05 eq) were added. The reaction mixture was purged three times with nitrogen atmosphere, and then under nitrogen atmosphere, the reaction system was heated to 100 °C and stirred for 12 h. After the reaction was completed, the reaction solution was cooled to 15 °C. Dioxane (5 mL) was then added, and the mixture was filtered. The filtrate was concentrated to give a crude product, and then ethyl acetate (5 mL) and *n*-heptane (5 mL) were added. The mixture was filtered and concentrated under reduced pressure to give compound **2-6.** MS (ESI) m/z: 373.1 [M+H⁺].

Step E: Compound **2-6** (0.436 g, 1.17 mmol, 1 eq) and compound **1-12A** (0.602 g, 2.27 mmol, 1.94 eq, dihydrochloride) were dissolved in *N*-methylpyrrolidinone (7 mL), and potassium carbonate (1.2 g, 8.68 mmol, 7.43 eq) was added. The reaction mixture was heated to 80 °C and stirred for 2 h. After the reaction was completed, the reaction solution was cooled to 15 °C, and water (20 mL) was added. The mixture was extracted with ethyl acetate (20 mL × 2) to give an organic phase, which was then dried over anhydrous sodium sulfate, filtered, and concentrated under reduced pressure to give compound **2-7.** MS (ESI) m/z: 529.3 [M+H⁺].

Step F: Compound **2-7** (0.46 g, 869.47 µmol, 1 eq) was dissolved in tetrahydrofuran (5 mL), and diisobutylaluminum hydride (1 mol/L, 3.48 mL, 4 eq) was added at 0 °C under nitrogen atmosphere. The reaction mixture was stirred at 15 °C for 1 h, and a saturated sodium sulfate solution (10 mL) was added to quench the reaction. Tetrahydrofuran (30 mL) was then added, and the mixture was filtered. The filtrate was concentrated under reduced pressure to give a crude product, which was then separated and purified by preparative high performance liquid chromatography (column model: Waters Xbridge BEH C18 (150 mm × 50 mm × 10 µm); mobile phase: [aqueous ammonium bicarbonate solution (10 mmol/L)-acetonitrile]; gradient: 22%-52%) to give **compound 2.** MS (ESI) m/z: 487.2 [M+H⁺]. ¹H NMR (400 MHz, DMSO-d6) δ = 7.74 (d, J = 5.2 Hz, 1H), 7.40 (s, 1H), 6.59 (d, J = 4.4 Hz, 1H), 6.05 (s, 1H), 5.74 (d, J = 5.6 Hz, 1H), 5.45 (br s, 1H), 4.49 (s, 2H), 4.15 (d, J= 10.8 Hz, 1H), 3.99 - 3.82 (m, 4H), 3.25 - 3.19 (m, 2H), 2.84 (d, J = 4.0 Hz, 3H), 2.40 (s, 3H), 1.98-1.92 (m, 1H), 1.80 - 1.75 (m, 1H), 1.68 - 1.65 (m, 1H), 1.55-1.52 (m, 1H).

### Example 3: Compound 3

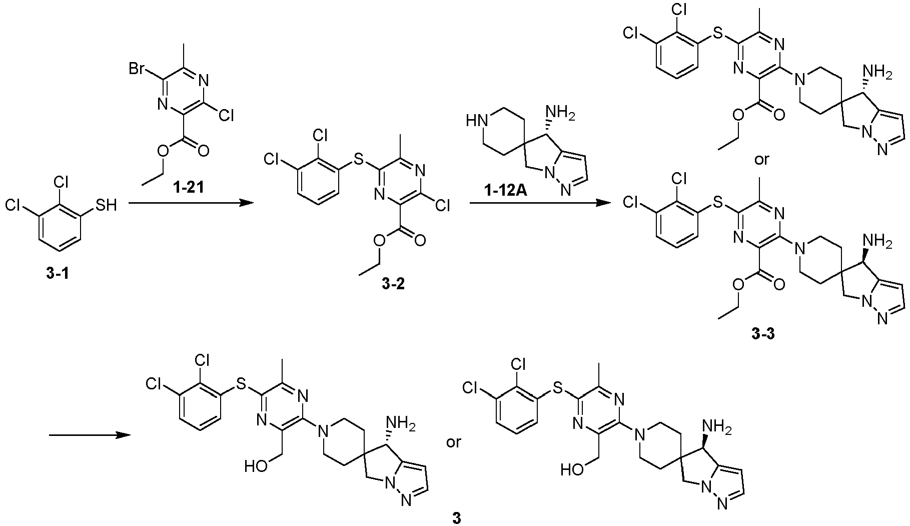

Step A: Compound **3-1** (500 mg, 2.79 mmol, 1.1 eq) and compound **1-21** (709.53 mg, 2.54 mmol, 1 eq) were dissolved in dioxane (10 mL), and *N*,*N*-diisopropylethylamine (984.22 mg, 7.62 mmol, 1.33 mL, 3 eq), 4,5-bis(diphenylphosphino)-9,9-dimethylxanthene (146.88 mg, 253.84 µmol, 0.1 eq), and tris(dibenzylideneacetone)dipalladium (116.22 mg, 126.92 µmol, 0.05 eq) were added. The reaction mixture was purged three times with nitrogen atmosphere, and then under nitrogen atmosphere, the reaction system was heated to 100 °C and stirred for 12 h. After the reaction was completed, ethyl acetate (10 mL) was added to the reaction solution, and the mixture was filtered. The filtrate was concentrated to give a crude product, which was then purified by silica gel thin layer chromatography (petroleum ether/ethyl acetate = 10:1) to give compound **3-2.** MS (ESI) m/z: 379.0 [M+H⁺].

Step B: Compound **3-2** (460 mg, 1.22 mmol, 1 eq) and compound **1-12A** (419.88 mg, 1.58 mmol, 1.3 eq, dihydrochloride) were dissolved in *N*-methylpyrrolidinone (6 mL), and potassium carbonate (841.69 mg, 6.09 mmol, 5 eq) was added. The reaction mixture was heated to 80 °C and stirred for 12 h. After the reaction was completed, water (10 mL) was added, and the mixture was extracted with ethyl acetate (10 mL × 3). The organic phases were combined, washed with saturated brine (10 mL × 2), dried over anhydrous sodium sulfate, and filtered. The filtrate was concentrated under reduced pressure to give a crude product of compound **3-3.** MS (ESI) m/z: 533.3 [M+H⁺].

Step C: Compound **3-3** (572 mg, 1.07 mmol, 1 eq) was dissolved in tetrahydrofuran (5 mL), and diisobutylaluminum hydride (1 mol/L, 4.29 mL, 4 eq) was added at 0 °C under nitrogen atmosphere. The reaction mixture was stirred at 25 °C for 0.5 h, and a saturated sodium sulfate solution (10 mL) was added to quench the reaction until no bubbles emerged. The mixture was then stirred at 25 °C for 30 min and filtered. The filtrate was concentrated under reduced pressure to give a crude product, which was then separated and purified by preparative high performance liquid chromatography (column model: Waters Xbridge C18 (150 mm × 50 mm × 10 µm); mobile phase [aqueous ammonium bicarbonate solution (10 mmol/L)-acetonitrile]; gradient: 36%-66%) to give **compound 3.** MS (ESI) m/z: 491.1 [M+H⁺]. ¹H NMR (400 MHz, DMSO-d6) δ = 7.49 (dd, J = 1.2, 8.0 Hz, 1H), 7.40 (d, J = 1.6 Hz, 1H), 7.26 (t, J = 8.0 Hz, 1H), 6.80 (dd, J = 1.6, 8.4 Hz, 1H), 6.05 (d, J = 1.6 Hz, 1H), 5.36 (t, J = 5.6 Hz, 1H), 4.47 (d, J = 5.6 Hz, 2H), 4.15 (d, J = 11.2 Hz, 1H), 4.02 - 3.92 (m, 2H), 3.90 - 3.77 (m, 2H), 3.25 - 3.16 (m, 2H), 2.43 (s, 3H), 2.02 - 1.86 (m, 3H), 1.82 - 1.72 (m, 1H), 1.71 - 1.63 (m, 1H), 1.53 (br d, J = 13.6 Hz, 1H).

### Example 4: Compound 4

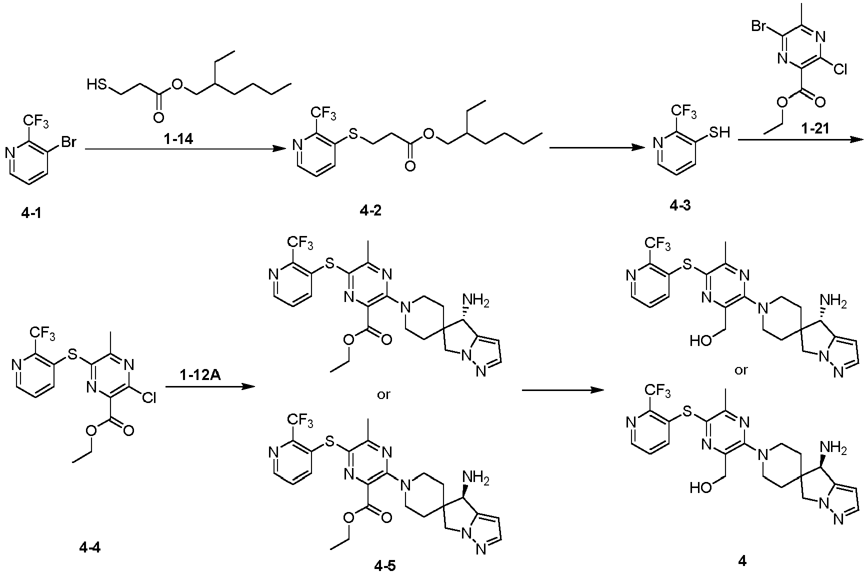

Step A: *N,N*-Diisopropylethylamine (3.43 g, 26.55 mmol, 4.62 mL, 3 eq), 4,5-bis(diphenylphosphino)-9,9-dimethylxanthene (512.07 mg, 884.98 µmol, 0.1 eq), and tris(dibenzylideneacetone)dipalladium (405.20 mg, 442.49 µmol, 0.05 eq) were added to a solution of compounds **4-1** (2 g, 8.85 mmol, 1 eq) and **1-14** (2.32 g, 10.62 mmol, 1.2 eq) in 1,4-dioxane (20 mL). The mixture was purged 3 times with nitrogen and then stirred at 100 °C for 12 h. After the reaction was completed, the mixture was cooled to room temperature and filtered. The filtrate was concentrated to give a crude product, which was then separated and purified by silica gel column chromatography (eluent: petroleum ether/ethyl acetate = 1:0 to 5:1) to give compound **4-2.** MS (ESI) m/z: 364.2 [M+H⁺].

Step B: A solution of sodium methoxide in methanol (5.4 mol/L, 713.35 µL, 30% purity, 2 eq) was added to a solution of compound **4-2** (700 mg, 1.93 mmol, 1 eq) in tetrahydrofuran (5 mL). The mixture was stirred at 25 °C for 30 min, adjusted to pH 7-8 with hydrogen chloride in ethyl acetate (4 mol/L), and then concentrated to give compound **4-3,** which was used directly in the next step.

Step C: *N,N*-Diisopropylethylamine (746.79 mg, 5.78 mmol, 1.01 mL, 3 eq), 4,5-bis(diphenylphosphino)-9,9-dimethylxanthene (111.45 mg, 192.61 µmol, 0.1 eq), and tris(dibenzylideneacetone)dipalladium (88.19 mg, 96.30 µmol, 0.05 eq) were added to a solution of compound **4-3** (345.08 mg, 1.93 mmol, 1 eq) and compound **1-21** (538.37 mg, 1.93 mmol, 1 eq) in 1,4-dioxane (7 mL). The mixture was purged 3 times with nitrogen and then stirred at 100 °C for 12 h. After the reaction was completed, the mixture was cooled to room temperature and filtered. The filtrate was concentrated to give a crude product, which was then separated and purified by silica gel column chromatography (eluent: petroleum ether/ethyl acetate = 1:0 to 5:1) to give compound **4-4.** MS (ESI) m/z: 378.1 [M+H⁺].

Step D: Potassium carbonate (384.14 mg, 2.78 mmol, 5 eq) was added to a solution of compound **4-4** (210 mg, 555.90 µmol, 1 eq) and compound **1-12A** (191.64 mg, 722.66 µmol, 1.3 eq, dihydrochloride) in *N,N*-dimethylformamide (5 mL). The mixture was stirred at 80 °C for 12 h. After the reaction was completed, the mixture was cooled to 25 °C and filtered. The filtrate was concentrated to give a crude product of compound **4-5.** MS (ESI) m/z: 534.2 [M+H⁺]. Step E: Diisobutylaluminum hydride (1 mol/L, 2.81 mL, 5 eq) was added dropwise to a solution of compound **4-5** (300 mg, 562.25 µmol, 1 eq) in tetrahydrofuran (3 mL) at 0 °C under nitrogen atmosphere. After the dropwise addition, the mixture was stirred at 25 °C for 1 h, and a saturated sodium sulfate solution was added to quench the reaction until no bubbles emerged from the reaction solution. The mixture was then stirred at 25 °C for 30 min and filtered. The filtrate was concentrated to give a crude product, which was then separated and purified by preparative high performance liquid chromatography (column model: Waters Xbridge 150 mm × 25 mm × 5 µm; mobile phase: [aqueous ammonia solution (0.05%)-acetonitrile]; gradient: 25%-55%) to give compound **4.** MS (ESI) m/z: 492.1 [M+H⁺]. ¹H NMR (400 MHz, DMSO-d6) δ = 8.56 (dd, J = 1.6, 4.4 Hz, 1H), 7.63 - 7.57 (m, 2H), 7.40 (d, J = 2.0 Hz, 1H), 6.05 (d, J = 1.6 Hz, 1H), 5.34 (t, J = 5.6 Hz, 1H), 4.44 (d, J = 5.6 Hz, 2H), 4.14 (d, J = 10.8 Hz, 1H), 3.98 - 3.94 (m, 2H), 3.85 - 3.76 (m, 2H), 3.22 - 3.15 (m, 2H), 2.42 (s, 3H), 2.03 (br s, 2H), 1.97 - 1.90 (m, 1H), 1.79 - 1.73 (m, 1H), 1.67 - 1.64 (m, 1H), 1.53-1.50 (m, 1H).

### Example 5: Compound 5

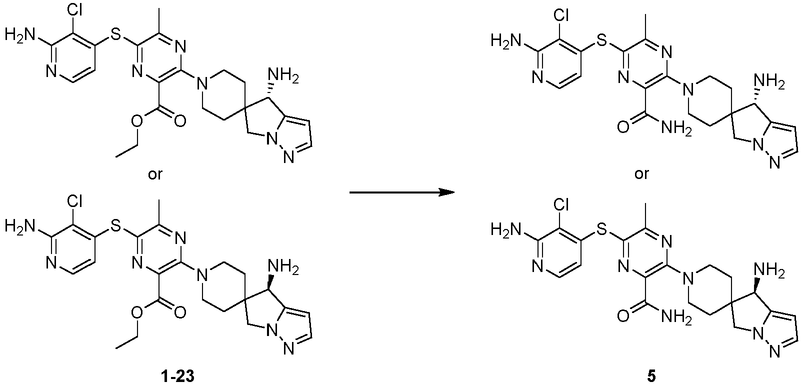

Step A: A solution of compound **1-23** (100 mg, 194.16 µmol, 1 eq) and ammonium hydroxide (1.13 g, 9.71 mmol, 1.25 mL, 30% purity, 50 eq) in 1,4-dioxane (2 mL) was heated to 80 °C in a sealed autoclave and stirred at 80 °C for 12 h. After the reaction was completed, the mixture was cooled to 25 °C and concentrated under reduced pressure to give a crude product, which was then separated and purified by preparative high performance liquid chromatography (column model: Waters Xbridge 150 mm × 25 mm × 5 µm; mobile phase: aqueous ammonia solution (0.05%)-acetonitrile; gradient: 15%-45%) to give compound **5.** MS (ESI) m/z: 486.1 [M+H⁺]. ¹H NMR (400 MHz, DMSO-d6) δ = 7.94 (s, 1H), 7.67 (d, J = 5.6 Hz, 1H), 7.60 (s, 1H), 7.41 (d, J = 2.0 Hz, 1H), 6.36 (s, 2H), 6.06 (d, J = 1.6 Hz, 1H), 5.77 (d, J = 5.2 Hz, 1H), 4.16 (d, J = 11.2 Hz, 1H), 4.05 - 3.87 (m, 4H), 3.30 - 3.27 (m, 2H), 2.41 (s, 3H), 1.92 - 1.86 (m, 1H), 1.73 - 1.61 (m, 2H), 1.54-1.50 (m, 1H).

### Example 6: Compound 6

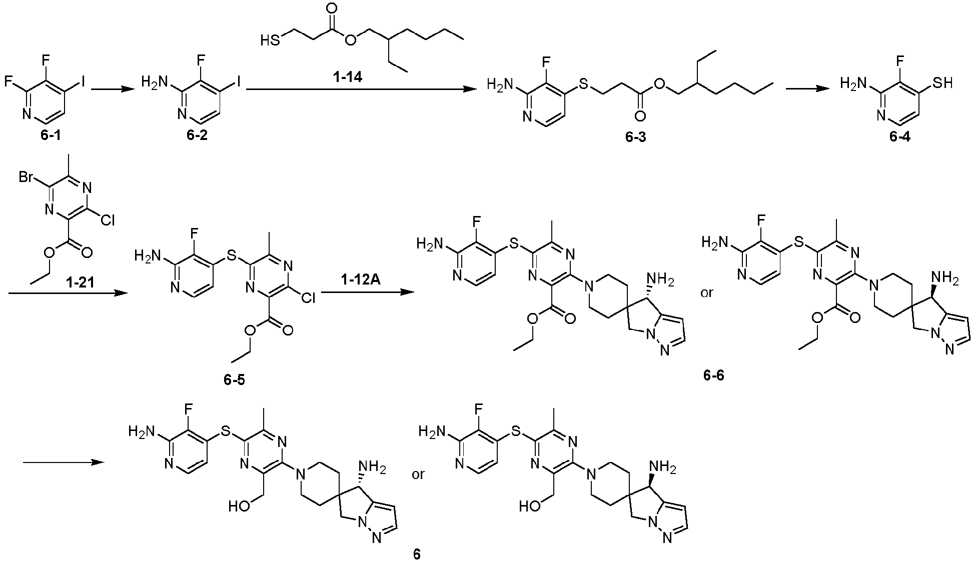

Step A: Compound **6-1** (3 g, 12.45 mmol, 1 eq) was dissolved in dioxane (15 mL), and ammonium hydroxide (13.65 g, 7.37 mmol, 15 mL, 25% purity, 7.92 eq) was added. The reaction solution was heated to 80 °C in a 100 mL tetrafluoro-sealed autoclave and stirred for 12 h. After the reaction was completed, the reaction solution was cooled to 25 °C. Water (60 mL) was then added to the reaction solution, and the mixture was stirred at 25 °C for 0.5 h and filtered. The filter cake was dried in vacuum to give compound **6-2.** MS (ESI) m/z: 239.0 [M+H⁺].

Step B: Compound **6-2** (2.3 g, 9.66 mmol, 1 eq) and compound **1-14** (2.53 g, 11.60 mmol, 1.2 eq) were dissolved in dioxane (25 mL), and *N,N*-diisopropylethylamine (3.75 g, 28.99 mmol, 5.05 mL, 3 eq), 4,5-bis(diphenylphosphino)-9,9-dimethylxanthene (1.12 g, 1.93 mmol, 0.2 eq), and tris(dibenzylideneacetone)dipalladium (884.94 mg, 966.38 µmol, 0.1 eq) were added. The reaction mixture was purged three times with nitrogen atmosphere, and then under nitrogen atmosphere, the reaction system was heated to 100 °C and stirred for 12 h. After the reaction was completed, ethyl acetate (10 mL) was added to the reaction solution, and the mixture was filtered. The filtrate was concentrated to give a crude product, and then *n*-heptane (40 mL) and ethyl acetate (2 mL) were added. The mixture was stirred at 25 °C for 0.5 h and filtered, and the filter cake was dried in vacuum to give compound **6-3.** MS (ESI) m/z: 329.3 [M+H⁺].

Step C: Compound **6-3** (500 mg, 1.52 mmol, 1 eq) was dissolved in tetrahydrofuran (5 mL), and a solution of sodium methoxide in methanol (5 mol/L, 395.81 µL, 1.3 eq) was added. The reaction mixture was stirred at 25 °C for 0.5 h. After the reaction was completed, the reaction solution was adjusted to pH 7 with hydrogen chloride in ethyl acetate (4 mol/L) and then directly concentrated to give compound **6-4.** MS (ESI) m/z: 145.1 [M+H⁺].

Step D: Compound **1-21** (386.83 mg, 1.38 mmol, 1 eq) and compound **6-4** (219.47 mg, 1.52 mmol, 1.1 eq) were dissolved in dioxane (5 mL), and *N*,*N*-diisopropylethylamine (536.57 mg, 4.15 mmol, 723.14 µL, 3 eq), 4,5-bis(diphenylphosphino)-9,9-dimethylxanthene (160.15 mg, 276.78 µmol, 0.2 eq), and tris(dibenzylideneacetone)dipalladium (126.73 mg, 138.39 µmol, 0.1 eq) were added. The reaction mixture was purged three times with nitrogen atmosphere, and then under nitrogen atmosphere, the reaction system was heated to 100 °C and stirred for 12 h. After the reaction was completed, ethyl acetate (10 mL) was added to the reaction solution, and the mixture was filtered. The filtrate was concentrated to give a crude product, which was then purified by silica gel thin layer chromatography (petroleum ether/ethyl acetate = 2:1) to give compound **6-5.** MS (ESI) m/z: 343.1 [M+H⁺].

Step E: Compound **6-5** (269 mg, 784.77 µmol, 1 eq) and compound **1-12A** (270.54 mg, 1.02 mmol, 1.3 eq, dihydrochloride) were dissolved in *N*-methylpyrrolidinone (5 mL), and potassium carbonate (542.31 mg, 3.92 mmol, 5 eq) was added. The reaction mixture was heated to 80 °C and stirred for 12 h. After the reaction was completed, water (10 mL) was added, and the mixture was extracted with ethyl acetate (10 mL × 3). The organic phases were combined, washed with saturated brine (10 mL × 2), dried over anhydrous sodium sulfate, and filtered. The filtrate was concentrated under reduced pressure to give a crude product, which was then separated and purified by preparative high performance liquid chromatography (column model: Waters Xbridge C18 150 mm × 50 mm × 10 µm; mobile phase: [aqueous ammonium bicarbonate solution (10 mmol/L)-acetonitrile ]; gradient B%: 22%-52%) to give compound **6-6.** MS (ESI) m/z: 499.1 [M+H⁺].

Step F: Compound **6-6** (215 mg, 431.23 µmol, 1 eq) was dissolved in tetrahydrofuran (5 mL), and diisobutylaluminum hydride (1 mol/L, 1.72 mL, 4 eq) was added at 0 °C under nitrogen atmosphere. The reaction mixture was stirred at 25 °C for 12 h, and a saturated sodium sulfate solution (10 mL) was added to quench the reaction until no bubbles emerged. The mixture was then stirred at 25 °C for 30 min and filtered. The filtrate was concentrated under reduced pressure to give a crude product, which was then separated and purified by preparative high performance liquid chromatography (column model: Waters Xbridge C18 150 mm × 50 mm × 10 µm; mobile phase [aqueous ammonium bicarbonate solution (10 mmol/L)-acetonitrile]; gradient: 15%-45%) to give compound **6.** MS (ESI) m/z: 457.1 [M+H⁺]. ¹H NMR (400 MHz, DMSO-d6) δ = 7.56 (d, J = 5.6 Hz, 1H), 7.40 (d, J = 1.6 Hz, 1H), 6.28 (s, 2H), 6.05 (d, J = 1.6 Hz, 1H), 5.93 (t, J = 5.2 Hz, 1H), 5.43 (t, J = 5.6 Hz, 1H), 4.47 (d, J = 5.6 Hz, 2H), 4.15 (d, J = 11.2 Hz, 1H), 4.02 - 3.91 (m, 2H), 3.89 - 3.76 (m, 2H), 3.25 - 3.14 (m, 2H), 2.47 - 2.39 (m, 3H), 2.01 - 1.87 (m, 1H), 1.82 - 1.71 (m, 1H), 1.71 - 1.62 (m, 1H), 1.52 (br d, J = 13.6 Hz, 1H).

### Example 7: Compound 7

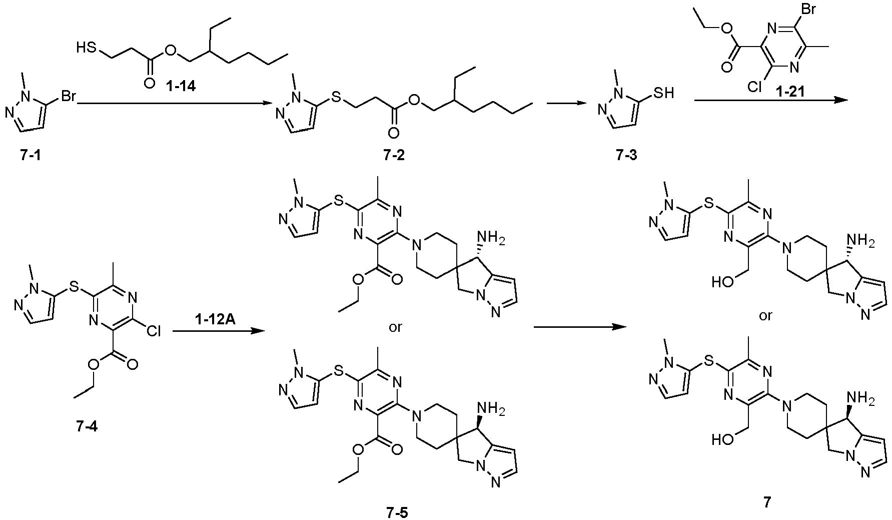

Step A: Compound **7-1** (3 g, 18.63 mmol, 1 eq) and compound **1-14** (4.88 g, 22.36 mmol, 1.2 eq) were dissolved in dioxane (30 mL), and *N,N*-diisopropylethylamine (4.82 g, 37.27 mmol, 6.49 mL, 2 eq), 4,5-bis(diphenylphosphino)-9,9-dimethylxanthene (539.09 mg, 931.68 µmol, 0.05 eq), and palladium acetate (209.17 mg, 931.68 µmol, 0.05 eq) were added. The reaction mixture was purged three times with nitrogen atmosphere, and then under nitrogen atmosphere, the reaction system was heated to 100 °C and stirred for 12 h, and then stirred at 120 °C for another 12 h. After the reaction was completed, the reaction solution was cooled to 15 °C, and methanol (30 mL) was added. The mixture was filtered, and the filtrate was concentrated to give a crude product, which was then subjected to column chromatography (silica gel, petroleum ether:ethyl acetate = 1:0 to 30:1) to give compound **7-2.** MS (ESI) m/z: 299.3 [M+H⁺].

Step B: Compound **7-2** (0.6 g, 2.01 mmol, 1 eq) was dissolved in tetrahydrofuran (6 mL), and a solution of sodium methoxide in methanol (5.4 mol/L, 744.60 µL, 2 eq) was added. The reaction mixture was stirred at 15 °C for 0.5 h. After the reaction was completed, the reaction solution was adjusted to pH 7 with a 4 mol/L solution of hydrogen chloride in ethyl acetate and then concentrated under reduced pressure to give a crude product of compound **7-3,** which was used directly in the next step. MS (ESI) m/z: 115.2 [M+H⁺].

Step C: Compound **7-3** (229.53 mg, 2.01 mmol, 1.1 eq) and compound **1-21** (510.87 mg, 1.83 mmol, 1 eq) were dissolved in dioxane (5 mL), and *N*,*N*-diisopropylethylamine (708.63 mg, 5.48 mmol, 955.02 mL, 3 eq), 4,5-bis(diphenylphosphino)-9,9-dimethylxanthene (105.75 mg, 182.77 µmol, 0.1 eq), and tris(dibenzylideneacetone)dipalladium (83.68 mg, 91.38 µmol, 0.05 eq) were added. The reaction mixture was purged three times with nitrogen atmosphere, and then under nitrogen atmosphere, the reaction system was heated to 100 °C and stirred for 12 h. After the reaction was completed, the reaction solution was cooled to 15 °C, and dioxane (5 mL) was added. The mixture was filtered, and the filtrate was concentrated to give a crude product, which was then subjected to column chromatography (silica gel, petroleum ether:ethyl acetate = 1:0 to 10:1) to give compound **7-4.** MS (ESI) m/z: 313.1 [M+H⁺].

Step D: Compound **7-4** (233 mg, 744.95 µmol, 1 eq) and compound **1-12A** (256.81 mg, 968.43 µmol, 1.3 eq, dihydrochloride) were dissolved in *N*,*N*-dimethylformamide (5 mL), and potassium carbonate (514.79 mg, 3.72 mmol, 5 eq) was added. The reaction mixture was heated to 80 °C and stirred for 12 h. After the reaction was completed, the reaction solution was cooled to 15 °C, filtered, and concentrated under reduced pressure to give compound **7-5.** MS (ESI) m/z: 469.3 [M+H⁺].

Step E: Compound **7-5** (307 mg, 655.18 µmol, 1 eq) was dissolved in tetrahydrofuran (3 mL), and diisobutylaluminum hydride (1 mol/L, 3.28 mL, 5 eq) was added at 0 °C under nitrogen atmosphere. The reaction mixture was stirred at 15 °C for 1 h, and a saturated sodium sulfate solution (10 mL) was added to quench the reaction. Tetrahydrofuran (20 mL) was then added, and the mixture was filtered. The filtrate was concentrated under reduced pressure to give a crude product, which was then separated and purified by preparative high performance liquid chromatography (column model: Waters Xbridge BEH C18 150 mm × 25 mm × 5 µm; mobile phase: [aqueous ammonia solution (0.05%)-acetonitrile]; gradient: 20%-50%) to give compound 7. MS (ESI) m/z: 427.3 [M+H⁺]. ¹H NMR (400 MHz,CDCl₃) δ = 7.60 (d, J = 2 Hz, 1H), 7.52 (d, J = 1.6 Hz, 1H), 6.52 (d, J = 2 Hz, 1H), 6.12 (d, J = 1.6 Hz, 1H),4.53 (s, 2H), 4.19 (d, J = 11.2 Hz, 1H), 4.08 (s, 1H), 4.01 (d, J = 10.8 Hz, 1H), 3.89 (s, 3H), 3.41-3.33 (m, 2H), 3.06 - 2.982 (m, 2H), 2.55 (s, 3H), 2.04 - 1.97 (m, 1H), 1.92 - 1.85 (m, 1H), 1.76 - 1.60 (m, 2H).

### Example 8: Compound 8

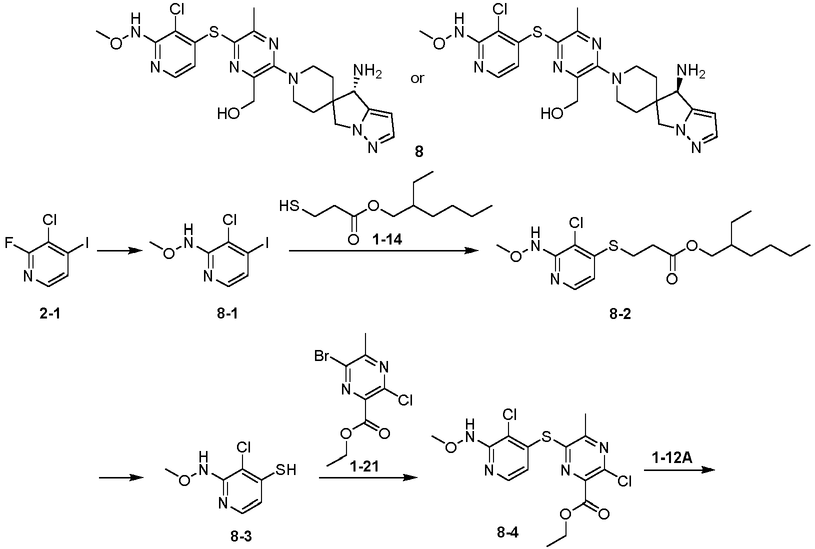

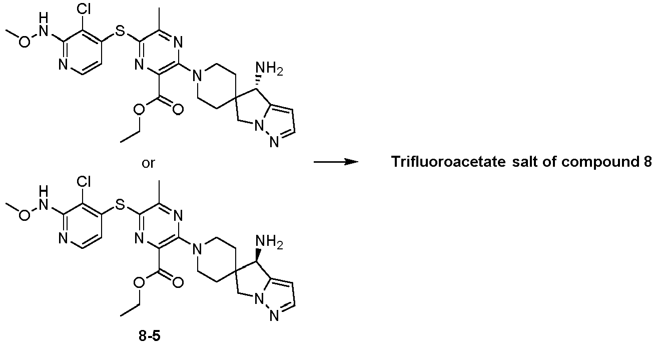

Step A: Compound **2-1** (1 g, 3.88 mmol, 1 eq), methoxyamine (1.62 g, 19.42 mmol, 5 eq, hydrochloride), and *N,N-*diisopropylethylamine (3.01 g, 23.31 mmol, 4.06 mL, 6 eq) were dissolved in dimethyl sulfoxide (15 mL), and the solution was microwaved for reaction at 100 °C for 12 h. After the reaction was completed, water (100 mL) and ethyl acetate (100 mL) were added. The organic phase was separated, and then the aqueous phase was extracted with ethyl acetate (100 mL). The organic phases were combined, washed with saturated brine (100 mL × 5), dried over anhydrous sodium sulfate, filtered, and concentrated under reduced pressure to give compound 8-1. MS (ESI) m/z: 285.0 [M+H⁺].

Step B: Compound **8-1** (2 g, 7.03 mmol, 1 eq), compound **1-14** (1.84 g, 8.44 mmol, 1.2 eq), *N,N-*diisopropylethylamine (1.82 g, 14.06 mmol, 2.45 mL, 2 eq), palladium acetate (78.92 mg, 351.52 µmol, 0.05 eq), and 4,5-bis(diphenylphosphino)-9,9-dimethylxanthene (203.39 mg, 351.52 µmol, 0.05 eq) were dissolved in dioxane (20 mL) under nitrogen atmosphere. The solution was allowed to react at 100 °C under nitrogen atmosphere for 3 h. After the reaction was completed, ethyl acetate (30 mL) was added to the reaction solution, and the mixture was filtered. The filtrate was concentrated under reduced pressure to give a crude product, which was then separated and purified by silica gel column chromatography (petroleum ether:ethyl acetate = 1:0 to 30:1) to give compound **8-2.** MS (ESI) m/z: 375.2 [M+H⁺].

Step C: Compound **8-2** (0.61 g, 1.63 mmol, 1 eq) was dissolved in tetrahydrofuran (6 mL) under nitrogen atmosphere, and a solution of potassium *tert*-butoxide in tetrahydrofuran (1 mol/L, 3.25 mL, 2 eq) was added. The mixture was stirred at 15 °C for 0.5 h. The reaction solution was adjusted to pH 7 with a 1 mol/L solution of hydrogen chloride in ethyl acetate and then concentrated under reduced pressure to give compound **8-3.** MS (ESI) m/z: 191.1 [M+H⁺]. Step D: Compound **8-3** (310.19 mg, 1.63 mmol, 1 eq), compound **1-21** (454.78 mg, 1.63 mmol, 1 eq), tris(dibenzylideneacetone)dipalladium (74.49 mg, 81.35 µmol, 0.05 eq), 4,5-bis(diphenylphosphino)-9,9-dimethylxanthene (94.14 mg, 162.70 µmol, 0.1 eq), and *N*,*N*-diisopropylethylamine (630.84 mg, 4.88 mmol, 850.19 µL, 3 eq) were dissolved in 1,4-dioxane (3 mL) under nitrogen atmosphere. The solution was allowed to react at 100 °C under nitrogen atmosphere for 12 h. After the reaction was completed, the solution was concentrated under reduced pressure to give a crude product, which was then separated and purified by silica gel column chromatography (petroleum ether:ethyl acetate = 1:0 to 10:1) to give compound **8-4.** MS (ESI) m/z: 389.0 [M+H⁺].

Step E: Compound **8-4** (80 mg, 205.52 µmol, 1 eq), compound **1-12A** (70.85 mg, 267.18 µmol, 1.3 eq, dihydrochloride), and *N*,*N*-diisopropylethylamine (159.37 mg, 1.23 mmol, 214.79 µL, 6 eq) were dissolved in *N,N-*dimethylformamide (1 mL). The solution was allowed to react at 80 °C for 1.5 h, cooled to room temperature, and filtered. The filtrate was concentrated under reduced pressure to give a crude product, which was then separated and purified by thin layer chromatography (dichloromethane:ethanol = 8:1) to give compound **8-5.** MS (ESI) m/z: 545.3 [M+H⁺].

Step F: Compound **8-5** (27 mg, 49.54 µmol, 1 eq) was dissolved in tetrahydrofuran (1 mL) under nitrogen atmosphere, and the solution was cooled to 0 °C. Diisobutylaluminum hydride (1 mol/L, 247.68 µL, 5 eq) was added dropwise, with the temperature maintained at no higher than 3 °C. After the dropwise addition, the mixture was stirred at 15 °C for 1 h. After the reaction was completed, a saturated sodium sulfate solution was added dropwise until no bubbles were generated in the reaction solution, and then tetrahydrofuran (10 mL) was added. The mixture was filtered in vacuum, and the filtrate was concentrated under reduced pressure to give a crude product, which was then separated and purified by preparative high performance liquid chromatography (column model: Phenomenex Synergi Polar-RP 100 mm × 25 mm × 4 µm; mobile phase: aqueous trifluoroacetic acid solution (0.1%)-acetonitrile; gradient: 14%-34%) to give a **trifluoroacetate salt of compound 8.** MS (ESI) m/z: 503.3 [M+H⁺]. ¹H NMR (400 MHz, DMSO-d₆) δ = 8.53 (brs, 3H), 7.65 (brs, 1H), 7.58 (s, 1H), 6.31 (s, 1H), 5.84 (brs, 1H), 4.49 (brs, 3H), 4.36 (d, J = 11.2 Hz, 1H), 4.22 (d, J = 11.2 Hz, 1H), 4.04 (d, J = 13.6 Hz, 1H), 3.94 (d, J = 13.6 Hz, 1H), 3.71 (s, 3H), 3.31-3.25 (m, 1H), 3.16 - 3.11 (m, 1H), 2.43 (s, 3H), 2.07 - 1.97 (m, 1H), 1.91 - 1.84 (m, 2H), 1.69 (d, J = 12.4 Hz, 1H).

### Example 9: Compound 9

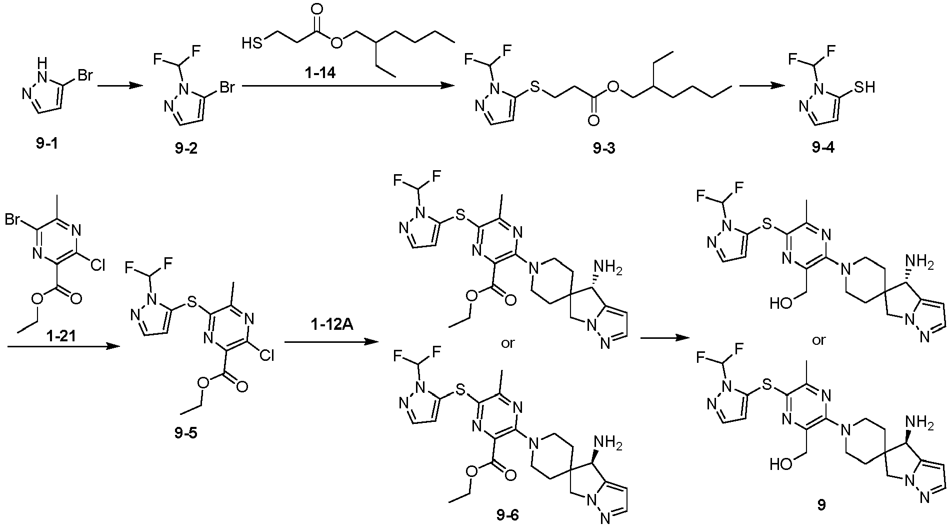

Step A: Compound **9-1** (10 g, 68.04 mmol, 1 eq) was dissolved in acetonitrile (100 mL), and anhydrous potassium fluoride (7.91 g, 136.08 mmol, 3.19 mL, 2 eq) and diethyl (bromodifluoromethyl)phosphonate (18.17 g, 68.04 mmol, 1 eq) were added. The reaction solution was stirred at 25 °C for 12 h. After the reaction was completed, acetonitrile (20 mL) was added to the reaction solution, and the mixture was filtered. The filtrate was concentrated under reduced pressure to give compound **9-2.** MS (ESI) m/z: 197.0,199.0 [M+H⁺].

Step B: Compound **9-2** (13.30 g, 60.92 mmol, 1.2 eq) and compound **1-14** (10 g, 50.77 mmol, 1 eq) were dissolved in dioxane (100 mL), and *N*,*N*-diisopropylethylamine (19.68 g, 152.30 mmol, 26.53 mL, 3 eq), 4,5-bis(diphenylphosphino)-9,9-dimethylxanthene (2.94 g, 5.08 mmol, 0.1 eq), and tris(dibenzylideneacetone)dipalladium (2.32 g, 2.54 mmol, 0.05 eq) were added. The reaction mixture was purged three times with nitrogen atmosphere, and then under nitrogen atmosphere, the reaction system was heated to 100 °C and stirred for 12 h. After the reaction was completed, ethyl acetate (10 mL) was added to the reaction solution, and the mixture was filtered. The filtrate was concentrated to give a crude product, which was then purified by silica gel thin layer chromatography (petroleum ether/ethyl acetate = 5:1) to give compound **9-3.** MS (ESI) m/z: 335.2 [M+H⁺]. Step C: Compound **9-3** (1 g, 2.99 mmol, 1 eq) was dissolved in tetrahydrofuran (10 mL), and a solution of sodium methoxide in methanol (5 mol/L, 777.46 µL, 1.3 eq) was added. The reaction mixture was stirred at 25 °C for 0.5 h. After the reaction was completed, the reaction solution was adjusted to pH 7 with hydrogen chloride/ethyl acetate (4 mol/L) and then directly concentrated to give compound **9-4.** MS (ESI) m/z: 151.1 [M+H⁺].

Step D: Compound **1-21** (759.84 mg, 2.72 mmol, 1 eq) and compound **9-4** (448.98 mg, 2.99 mmol, 1.1 eq) were dissolved in dioxane (10 mL), and *N*,*N*-diisopropylethylamine (1.05 g, 8.16 mmol, 1.42 mL, 3 eq), 4,5-bis(diphenylphosphino)-9,9-dimethylxanthene (157.29 mg, 271.84 µmol, 0.1 eq), and tris(dibenzylideneacetone)dipalladium (124.46 mg, 135.92 µmol, 0.05 eq) were added. The reaction mixture was purged three times with nitrogen atmosphere, and then under nitrogen atmosphere, the reaction system was heated to 100 °C and stirred for 12 h. After the reaction was completed, ethyl acetate (10 mL) was added to the reaction solution, and the mixture was filtered. The filtrate was concentrated to give a crude product, which was then purified by silica gel thin layer chromatography (petroleum ether/ethyl acetate = 3:1) to give compound 9-5. MS (ESI) m/z: 349.0 [M+H⁺].

Step E: Compound **9-5** (400 mg, 1.15 mmol, 1 eq) and compound **1-12A** (395.39 mg, 1.49 mmol, 1.3 eq, dihydrochloride) were dissolved in *N*,*N*-dimethylformamide (5 mL), and potassium carbonate (792.57 mg, 5.73 mmol, 5 eq) was added. The reaction mixture was heated to 80 °C and stirred for 12 h. After the reaction was completed, the mixture was filtered, and the filtrate was concentrated under reduced pressure to give compound **9-6.** MS (ESI) m/z: 505.2 [M+H⁺].

Step F: Compound **9-6** (0.7 g, 1.39 mmol, 1 eq) was dissolved in tetrahydrofuran (7 mL), and diisobutylaluminum hydride (1 mol/L, 6.94 mL, 5 eq) was added at 0 °C under nitrogen atmosphere. The reaction mixture was stirred at 25 °C for 0.5 h, and a saturated sodium sulfate solution (10 mL) was added to quench the reaction until no bubbles emerged. The mixture was then stirred at 25 °C for 30 min and filtered. The filtrate was concentrated under reduced pressure to give a crude product, which was then separated and purified by preparative high performance liquid chromatography (column: Waters Xbridge C18 150 mm × 50 mm × 10 µm; mobile phase [aqueous ammonium bicarbonate solution (10 mmol/L)-acetonitrile]; B%: 22%-52%, 10 min) to give compound 9. MS (ESI) m/z: 463.1 [M+H⁺]. ¹H NMR (400 MHz, DMSO-d₆) δ = 8.17 - 7.74 (m, 2H), 7.38 (s, 1H), 6.73 (s, 1H), 6.03 (d, J = 1.2 Hz, 1H), 5.08 (t, J = 5.6 Hz, 1H), 4.40 (d, J = 5.6 Hz, 2H), 4.11 (d, J = 11.2 Hz, 1H), 3.98 - 3.87 (m, 2H), 3.78 - 3.59 (m, 2H), 3.16 - 3.02 (m, 2H), 2.48 (br s, 3H), 2.03 - 1.83 (m, 3H), 1.78 - 1.68 (m, 1H), 1.66 - 1.58 (m, 1H), 1.48 (br d, J = 13.2 Hz, 1H).

### Example 10: Compound 10

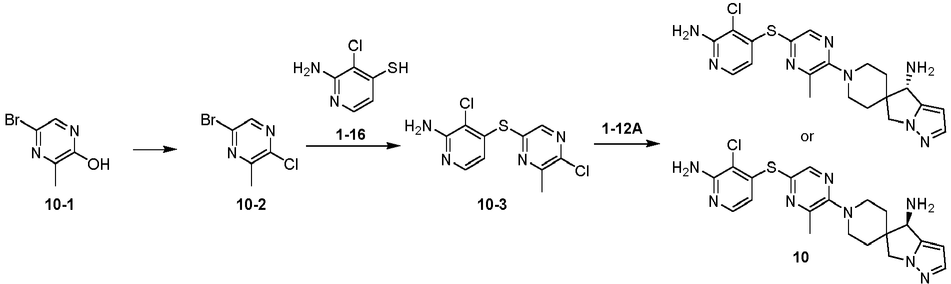

Step A: Phosphorus oxychloride (4.06 g, 26.45 mmol, 2.46 mL, 5 eq) was added to a solution of compound **10-1** (1 g, 5.29 mmol, 1 eq) in 1,4-dioxane (15 mL). The mixture was stirred under nitrogen atmosphere at 90 °C for 12 h and then cooled to 25 °C. Water (50 mL) was added dropwise to quench the reaction, and the mixture was extracted with ethyl acetate (50 mL × 2). The organic phases were combined, washed with brine (50 mL × 2), dried over anhydrous sodium sulfate, and concentrated under reduced pressure to give a crude product of compound **10-2,** which was used directly in the next step.

Step B: *N*,*N*-Diisopropylethylamine (1.50 g, 11.57 mmol, 2.02 mL, 3 eq), 4,5-bis(diphenylphosphino)-9,9-dimethylxanthene (446.26 mg, 771.25 µmol, 0.2 eq), and tris(dibenzylideneacetone)dipalladium (353.12 mg, 385.62 µmol, 0.1 eq) were added to a solution of compound **10-2** (800 mg, 3.86 mmol, 1 eq) and compound **1-16** (619.41 mg, 3.86 mmol, 1 eq) in 1,4-dioxane (10 mL). The mixture was purged 3 times with nitrogen and then stirred at 90 °C for 12 h. After the reaction was completed, the mixture was cooled to room temperature and filtered. The filtrate was concentrated to give a crude product, which was then separated and purified by preparative high performance liquid chromatography (column model: Phenomenex luna C18 250 mm × 50 mm × 10 µm; mobile phase: aqueous hydrochloric acid solution (0.05%)-acetonitrile; gradient: 30%-60%) to give compound **10-3.** MS (ESI) m/z: 287.0 [M+H⁺].

Step C: Potassium carbonate (24.06 mg, 174.11 µmol, 5 eq) was added to a solution of compound **10-3** (10 mg, 34.82 µmol, 1 eq) and compound **1-12A** (9.23 mg, 34.82 µmol, 1 eq, dihydrochloride) in *N*,*N*-dimethylformamide (1 mL). The mixture was stirred at 100 °C for 12 h, then cooled to room temperature, and filtered. The filtrate was concentrated under reduced pressure to give a crude product, which was then separated and purified by preparative high performance liquid chromatography (column model: Waters Xbridge 150 mm × 25 mm × 5 µm; mobile phase

[ammonium hydroxide (0.05%)-acetonitrile ]; gradient: 19%-49%) to give compound **10.** MS (ESI) m/z: 443.3 [M+H⁺]. ¹H NMR (400 MHz, DMSO-d₆) δ = 8.30 (s, 1H), 7.67 (d, J = 5.6 Hz, 1H), 7.44 (s, 1H), 6.37 (s, 2H), 6.10 (s, 1H), 5.89 (d, J = 5.2 Hz, 1H), 4.18 - 4.01 (m, 3H), 3.67 - 3.59 (m, 2H), 3.19 - 3.07 (m, 2H), 2.50 (s, 3H), 2.01 - 1.93 (m, 1H), 1.84 - 1.75 (m, 1H), 1.71 - 1.57 (m, 2H).

### Experimental Example 1: SHP2 In-Vitro Enzymology Experiment

**Experimental materials:** Homogeneous Full Length SHP-2 Assay Kit (purchased from BPS Bioscience) and NIVO multimode microplate reader.

### Methodology:

Preparation of 1× buffer (ready-to-use): 5× buffer was diluted with deionized water to give 1× buffer, which was then placed on ice for later use.

Each test compound was diluted with 100% DMSO to 100 µM as the first concentration, and then diluted in a 4-fold gradient to the 8^{th} concentration (i.e., from 100 µM to 6.1 nM) using a multi-channel pipette. Each gradient of the test compound was diluted with 1× buffer to prepare a working solution with 10% DMSO. The resulting solution was then added to the corresponding wells of a plate at 5 µL/well, with duplicate wells set up. The plate was then centrifuged at 1000 rpm for 1 min. 18 µL of a prepared reaction mixed solution (containing 12.25 µL of deionized water, 5 µL of 5× buffer; 0.25 µL of SHP-2 substrate polypeptide (100 µM), and 0.5 µL of DTT (250 mM)) was added to each well. The plate was then centrifuged at 1000 rpm for 1 min.

An SHP-2 enzyme was diluted to 0.1 ng/µL with 1× buffer. The resulting solution was added to the corresponding wells at 2 µL/well, and 2 µL of 1× buffer and SHP-2 (0.2 ng) were added to each negative control well. This step was performed on ice. The reaction system was then incubated at 25 °C for 60 min for compound pre-incubation. After the compound pre-incubation was completed, 25 µL of a substrate working solution (containing 19.45 µL of deionized water, 5 µL of 5× buffer; 0.5 µL of DTT (250 mM), and 0.05 µL of SHP-2 Substrate (DiFMUP) (10 mM)) was added to each well, and the reaction system was allowed to react at 25 °C for 30 min. At that time, the final concentration gradient of the compound ranged from 1 µM to 0.061 nM. After the reaction was completed, the fluorescence values were read using the NIVO multimode microplate reader, with an excitation wavelength of 360 nm and an emission wavelength of 460 nm.

Detection of compound background reading values: Each gradient of the test compound diluted with 100% DMSO was added to a new compound plate at 5 µL/well, and then 1× buffer was added at 45 µL/well to achieve a 10-fold dilution to prepare a working solution with 10% DMSO. Next, the compound working solution was added to the detection plate at 5 µL/well, and then 1× buffer was added at 45 µL/well to achieve another 10-fold dilution, bringing the final concentration of DMSO to 1%. The plate was then centrifuged at 1000 rpm for 1 min. The fluorescence values were read using the multimode microplate reader, with an excitation wavelength of 360 nm and an emission wavelength of 460 nm.

### Data analysis:

The original data were converted to inhibition rates and the IC₅₀ values were obtained by curve fitting using a four-parameter method. Table 1 illustrates the effects of the compounds disclosed herein on SHP2 enzyme activity. **Experimental results:** The results are shown in Table 1.

**Table 1**

| **Sample** | **SHP2 IC₅₀ (nmol/L)** |
|---|---|
| Compound **1** | 1.73 |
| Compound **2** | 2.87 |
| Compound **3** | 2.28 |
| Compound **4** | 2.14 |
| Compound **5** | 3.24 |
| Compound **6** | 3.15 |
| Compound **8** | 3.99 |
| Compound **10** | 1.09 |

**Conclusion:** The compounds disclosed herein had excellent *in-vitro* inhibitory activity against SHP2.

### Experimental Example 2: Anti-Proliferation Experimental Study on Tumor Cells with KRAS Mutations Experimental materials:

Cell culture medium, penicillin/streptomycin antibiotics purchased from Wisent, and fetal bovine serum purchased from Biosera; 3D CellTiter-Glo (chemiluminescence detection reagent for cell viability) reagent purchased from Promega; Cell lines purchased from Nanjing Cobioer Biosciences Co., Ltd. and Wuhan Procell Life Science&Technology Co., Ltd; and Envision multimode microplate reader (PerkinElmer).

### Methodology:

Different types of tumor cells were each seeded in an ultra-low attachment 96-well U-bottom plate, with 80 µL of cell suspension per well. The cell density conditions are shown in the table below. The cell plate was incubated in a CO₂ incubator overnight.

| Cell name | Plating density | Duration of incubation with compound (day) |
|---|---|---|
| MIAPACA2_PANCREAS | 1000 | 3 |
| NCIH358_LUNG | 2000 | 5 |

Each test compound was diluted in a 5-fold gradient to the 9^{th} concentration (i.e., from 2 mM to 5.12 nM or from 200 µM to 0.512 nM) using a multi-channel pipette, with duplicate wells set up. 78 µL of medium was added to an intermediate plate, and the serially diluted compound was transferred to the corresponding wells of the intermediate plate at 2 µL/well. The mixture was mixed well and then transferred to the cell plate at 20 µL/well. The concentration of the compound transferred to the cell plate ranged from 10 µM to 0.0256 nM or from 1 µM to 0.00256 nM. The cell plate was then incubated in a CO₂ incubator for 3-6 days. The specific duration is shown in Table 1.

The chemiluminescence detection reagent for cell viability was added to the cell plate at 100 µL/well, and the luminescence signals were stabilized by incubation for 10 min at room temperature. Readings were taken using the multimode microplate reader.

### Data analysis:

The original data were converted to inhibition rates and then the IC₅₀ values were obtained by curve fitting using a four-parameter method (obtained from the "log(inhibitor) vs. response -- Variable slope" model in GraphPad Prism). Table 2 illustrates the inhibitory activity of the compounds disclosed herein against the proliferation of different types of tumor cells.

**Experimental results:** The results are shown in Tables 2 and 3.

**Table 2. Inhibitory activity of compounds disclosed herein against H358 cell proliferation**

| **Sample** | **H358 anti-proliferation IC₅₀ (nmol/L)** |
|---|---|
| Compound **1** | 22.10 |
| Compound **2** | 61.43 |
| Compound **3** | 62.09 |
| Compound **4** | 144.80 |
| Compound **5** | 43.10 |
| Compound **6** | 120.20 |
| Compound **10** | 38.86 |

**Table 3. Inhibitory activity of compounds disclosed herein against MiaPaCa-2 cell proliferation**

| **Sample** | **MiaPaCa-2 anti-proliferation IC₅₀ (nmol/L)** |
|---|---|
| Compound **1** | 22.56 |
| Compound **2** | 24.40 |
| Compound **3** | 35.00 |
| Compound **4** | 56.90 |
| Compound **5** | 48.47 |
| Compound **6** | 62.15 |
| Compound **10** | 19.31 |

### Conclusion:

The compounds disclosed herein had good anti-proliferative inhibitory effects against H358 and MiaPaCa-2 cancer cells with KRAS mutations.

### Experimental Example 3: Study on Inhibitory Activity against ERK Phosphorylation in H358 Cells Experimental materials:

H358 cells purchased from Nanjing Cobioer Biosciences Co., Ltd., 1640 medium purchased from Biological industries, fetal bovine serum purchased from Biosera, and Advanced Phospho-ERK1/2 (THR202/TYR204) KIT purchased from Cisbio.

Composition of Advanced Phospho-ERK1/2 (THR202/TYR204) KIT:

| Component name | Storage temperature |
|---|---|
| Advanced PhosphoERK1/2 Eu Cryptate antibody | ≤-16°C |
| Advanced PhosphoERK1/2 d2 antibody | ≤-16°C |
| Blocking reagent (stock solution 100×) | ≤-16°C |
| Lysis buffer #1 (stock solution 4×) | ≤-16°C |
| Detection buffer (ready-to-use) | ≤-16°C |

### Methodology:

1. H358 cells were plated in a transparent 96-well cell culture plate, with 80 µL of cell suspension per well, each well containing 10000 H358 cells. The cell plate was incubated in a CO₂ incubator at 37 °C overnight.
2. The cell supernatant was discarded, and a starvation medium (1640 + 0.02% fetal bovine serum + 1% bispecific antibody) was added at 80 µL/well. The cell plate was then incubated in a CO₂ incubator for cell starvation treatment overnight;
3. Each test compound was diluted with 100% DMSO to 4 mM as the first concentration, and then diluted in a 5-fold gradient to the 8^{th} concentration (i.e., from 4 mM to 10.24 µM) using a pipette. 1 µL of the compound was then added to 79 µL of cell starvation medium. The mixture was mixed well, and then the compound solution was added to the corresponding wells of the cell plate at 20 µL/well. The cell plate was then put back to the CO₂ incubator for incubation for another 1 h. At that time, the concentration of the compound ranged from 10 µM to 0.0256 nM, with the concentration of DMSO being 0.25%.
4. After the incubation was completed, the cell supernatant was discarded, and a cell lysis buffer was added at 50 µL/well. The plate was then incubated with shaking at room temperature for 30 min.
5. The Phospho-ERK1/2 Eu Cryptate antibody and Phospho-ERK1/2 d2 antibody were diluted in a 20-fold gradient with the Detection buffer.
6. 16 µL of the cell lysate supernatant from each well was transferred to a new 3 84-well white microplate, and then 2 µL of the Phospho-ERK1/2 Eu Cryptate antibody dilution and 2 µL of the Phospho-ERK1/2 d2 antibody dilution were added to each well. The mixture was incubated at room temperature for 4 h.
7. After the incubation was completed, HTRF values were read using a multimode microplate reader, with an excitation wavelength of 320 nm and emission wavelengths of 615 nm and 665 nm.

### Data analysis:

The original data were converted to inhibition rates and then the IC₅₀ values were obtained by curve fitting using a four-parameter method.

**Experimental results:** The results are shown in Table 4.

**Table 4. Inhibitory activity of compounds disclosed herein against ERK phosphorylation in H358 cells**

| **Sample** | **H358 cell ERK phosphorylation IC₅₀ (nmol/L)** |
|---|---|
| Compound **1** | 21.24 |
| Compound **2** | 84.14 |
| Compound **3** | 55.62 |
| Compound **4** | 75.78 |
| Compound **5** | 45.26 |
| Compound **6** | 57.11 |

### Conclusion:

The compounds disclosed herein had strong inhibitory effects against ERK phosphorylation in H358 cancer cells with KRAS mutations.

### Experimental Example 4: In-Vivo Pharmacokinetic Study in Mice

### Experimental objective:

This experiment was intended to evaluate the pharmacokinetic behavior of each compound after a single intravenous injection and intragastric administration and to investigate the bioavailability after intragastric administration.

### Procedures:

Male CD-1 mice aged 7 to 10 weeks were selected. The mice were fasted for at least 12 h before administration and were allowed to feed again 4 h after administration, with free access to water throughout the experiment.

On the day of the experiment, animals in the intravenous group were given the corresponding compound by a single injection via the tail vein at an administration volume of 5 mL/kg; animals in the oral group were given the corresponding compound by a single intragastric administration at an administration volume of 10 mL/kg. The animals were weighed before administration, and the administration volume was calculated based on their body weight. Sample collection time points: For the injection group, samples were collected at 0.083 h, 0.25 h, 0.5 h, 1 h, 2 h, 4 h, 8 h, and 24 h; for the intragastric group, samples were collected at 0.25 h, 0.5 h, 1 h, 2 h, 4 h, 6 h, 8 h, and 24 h. At each time point, approximately 30 µL of whole blood was collected via the saphenous vein, which was then used to prepare plasma for concentration determination by high performance liquid chromatography-tandem mass spectrometry (LC-MS/MS). All animals were euthanized with CO₂ anesthesia after the PK samples were taken at the last time point. The plasma concentrations were processed using a non-compartmental model of the pharmacokinetic software WinNonlin^{™} Version 6.3 (Pharsight, Mountain View, CA), and the pharmacokinetic parameters were calculated using a linear log-trapezoidal method.

**Experimental results:** The evaluation results of PK properties are shown in Table 5.

### Conclusion:

The compounds disclosed herein had excellent pharmacokinetic properties.

**Table 5. Evaluation results of PK properties in mice**

| **Compound** | **Injection** | | | **Oral administration** | | | |
|---|---|---|---|---|---|---|---|
| | **Dose (mg/kg)** | **Cl (mL/min/kg)** | **AUC₀₋ₗₐₛₜ (nM.h)** | **Dose (mg/kg)** | **Cmax (nM)** | **AUC₀₋ₗₐₛₜ (nM.h)** | **F%** |
| Compound 1 | **2** | 27.7 | 2532 | **10** | 3537 | 8602 | 69.5 |
| Compound 2 | **1** | 27 | 1316 | **2** | 927 | 2035 | 83.6 |
| Compound 4 | **1** | 35.5 | 974 | **2** | 372 | 990 | 58.6 |
| Compound 6 | **1** | 68.3 | 638 | **2** | 463 | 817 | 89.8 |

### Conclusion:

The compounds disclosed herein showed excellent AUC and bioavailability after oral administration in mice, indicating good pharmacokinetic properties.

### Experimental Example 5: In-Vivo Pharmacokinetic Study in Rats

### Experimental objective:

This experiment was intended to evaluate the pharmacokinetic behavior of each compound after a single intravenous injection and intragastric administration and to investigate the bioavailability after intragastric administration.

### Procedures:

Male SD rats aged 7 to 10 weeks were selected. The rats were fasted for at least 12 h before administration and were allowed to feed again 4 h after administration, with free access to water throughout the experiment.

On the day of the experiment, animals in the intravenous group were given the corresponding compound by a single injection via the tail vein at an administration volume of 5 mL/kg; the oral group was given the corresponding compound by a single intragastric administration at an administration volume of 10 mL/kg. The animals were weighed before administration, and the administration volume was calculated based on their body weight. Sample collection time points: 0.083 h (injection group), 0.25 h, 0.5 h, 1 h, 2 h, 4 h, 6 h, 8 h, and 24 h. At each time point, approximately 200 µL of whole blood was collected via the jugular vein, which was then used to prepare plasma for concentration determination by high performance liquid chromatography-tandem mass spectrometry (LC-MS/MS). All animals were euthanized with CO₂ anesthesia after the PK samples were taken at the last time point. The plasma concentrations were processed using a non-compartmental model of the pharmacokinetic software WinNonlin^{™} Version 6.3 (Pharsight, Mountain View, CA), and the pharmacokinetic parameters were calculated using a linear log-trapezoidal method.

**Experimental results:** The evaluation results of *in-vivo* PK properties in rats are shown in Table 6.

**Table 6. Evaluation results of in-vivo PK properties in rats**

| **Compound** | **Injection** | | | **Oral administration** | | | |
|---|---|---|---|---|---|---|---|
| | **Dose (mg/kg)** | **Cl (mL/min/kg)** | **AUC₀₋ₗₐₛₜ (nM.h)** | **Dose (mg/kg)** | **Cmax (nM)** | **AUC₀₋ₗₐₛₜ (nM.h)** | **F%** |
| Compound 1 | **2** | 17.3 | 4050 | **20** | 9727 | 39709 | 98.0 |

### Conclusion:

The compounds disclosed herein showed excellent AUC and bioavailability after oral administration in rats, indicating good pharmacokinetic properties.

### Experimental Example 6: In-Vivo Experiment on Inhibition of MiaPaCa-2 Subcutaneous Xenograft Tumor

**Experimental objective:**

To evaluate the antitumor effect of each test drug in a female BALB/c nude mouse animal model with subcutaneous xenograft of a human pancreatic cancer MIA PaCa-2 cell line.

### Procedures:

MIA PaCa-2 cells were cultured in a DMEM medium containing 2.5% HS and 10% fetal bovine serum. MIA PaCa-2 cells in the logarithmic growth phase were collected and resuspended in PBS to achieve an appropriate concentration for subcutaneous tumor inoculation in nude mice.

The experimental mice were inoculated subcutaneously on the right dorsal side with 5 × 10⁶ MIA PaCa-2 cells (the cells were resuspended in a 1:1 mixture of PBS and matrigel), with 0.1 mL per mouse. Tumor growth was observed regularly. Once the tumors reached an average volume of approximately 125 (100-150) mm³, the mice were randomly divided into groups for administration based on the tumor size and body weight.

After the start of the administration, the body weight and tumor size of the mice were measured twice a week. Calculation formula for tumor volume: Tumor volume (mm³) = 1/2 × (a × b²) (where a represents long diameter and b represents short diameter).

**Experimental results:** The tumor inhibitory effects of the compounds are shown in Table 7.

**Table 7. Results of MiaPaCa-2 xenograft orthotopic transplantation experiment**

| **Compound** | | **Blank** | **Compound 1** | **Compound 2** |
|---|---|---|---|---|
| **Dose of administration (mg/kg)** | | 0 | 30 | 30 |
| **Frequency of administration** | | Once daily | Once daily | Once daily |
| **Mode of administration** | | Oral administration | Oral administration | Oral administration |
| **Mean tumor volume (mm³)** | **Day 12** | 129.22 | 129.20 | 129.17 |
| | **Day 39** | 512.12 | 278.34 | 248.09 |
| **Inhibition rate (%) at day 39** | | | 63.1 | 66.6 |

### Conclusion:

The compounds disclosed herein had significant inhibitory effects on the growth of MiaPaCa-2 subcutaneous xenograft tumors.

## Claims

1. A compound of formula (I'), a stereoisomer thereof, or a pharmaceutically acceptable salt thereof: 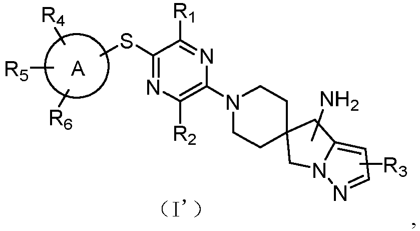 wherein
ring A is selected from the group consisting of aryl and heteroaryl;
R₁ is selected from the group consisting of H, deuterium, NH₂, and C₁₋₆ alkyl optionally substituted with one or more halogens, CN, or OH;
R₂ is selected from the group consisting of deuterium, H, halogen, OH, CN, COOH, -C(=O)-C₁₋₆ alkyl, -COO-C₁₋₆ alkyl, C₁₋₆ alkyl, and -C(=O)NH₂, wherein the -C(=O)-C₁₋₆ alkyl, -COO-C₁₋₆ alkyl, C₁₋₆ alkyl and -C(=O)NH₂ are each independently optionally substituted with 1, 2, or 3 Rₐ;
R₃ is selected from the group consisting of H, halogen, OH, NO₂, CN, C₁₋₆ alkyl, C₁₋₆ alkoxy, and C₁₋₆ alkylamino, wherein the C₁₋₆ alkyl, C₁₋₆ alkoxy and C₁₋₆ alkylamino are each independently optionally substituted with 1, 2, or 3 R_{b};
R₄, R₅, and R₆ are each independently selected from the group consisting of H, deuterium, halogen, NH₂, NO₂, CN, OH, C₁₋₆ alkyl, C₁₋₆ alkoxy, and -NH-O-C₁₋₆ alkyl, wherein the NH₂, C₁₋₆ alkyl, C₁₋₆ alkoxy and -NH-O-C₁₋₆ alkyl are each independently optionally substituted with 1, 2, or 3 R_{c};
Rₐ, R_{b}, and R_{c} are each independently selected from the group consisting of deuterium, halogen, OH, NH₂, and C₁₋₃ alkyl.

2. The compound, the stereoisomer thereof, or the pharmaceutically acceptable salt thereof according to claim 1, being selected from a compound of formula (I) or a pharmaceutically acceptable salt thereof: 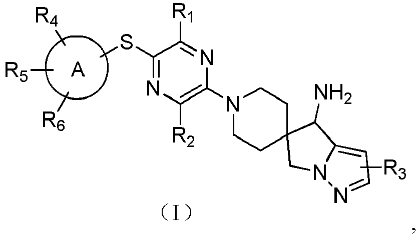 wherein
ring A is selected from the group consisting of aryl and heteroaryl;
R₁ is selected from the group consisting of H, deuterium, NH₂, and C₁₋₆ alkyl optionally substituted with one or more halogens, CN, or OH;
R₂ is selected from the group consisting of deuterium, H, halogen, OH, CN, COOH, -C(=O)-C₁₋₆ alkyl, -COO-C₁₋₆ alkyl, C₁₋₆ alkyl, and -C(=O)NH₂, wherein the -C(=O)-C₁₋₆ alkyl, -COO-C₁₋₆ alkyl, C₁₋₆ alkyl and -C(=O)NH₂ are each independently optionally substituted with 1, 2, or 3 Rₐ;
R₃ is selected from the group consisting of H, halogen, OH, NO₂, CN, C₁₋₆ alkyl, C₁₋₆ alkoxy, and C₁₋₆ alkylamino, wherein the C₁₋₆ alkyl, C₁₋₆ alkoxy and C₁₋₆ alkylamino are each independently optionally substituted with 1, 2, or 3 R_{b};
R₄, R₅, and R₆ are each independently selected from the group consisting of H, deuterium, halogen, NH₂, NO₂, CN, OH, C₁₋₆ alkyl, C₁₋₆ alkoxy, and -NH-O-C₁₋₆ alkyl, wherein the NH₂, C₁₋₆ alkyl, C₁₋₆ alkoxy and -NH-O-C₁₋₆ alkyl are each independently optionally substituted with 1, 2, or 3 R_{c};
Rₐ, R_{b}, and R_{c} are each independently selected from the group consisting of deuterium, halogen, OH, NH₂, and C₁₋₃ alkyl.

3. The compound, the stereoisomer thereof, or the pharmaceutically acceptable salt thereof according to claim 1 or 2, wherein
ring A is selected from the group consisting of aryl and 5- to 6-membered heteroaryl, wherein the 5- to 6-membered heteroaryl comprises 1, 2, or 3 heteroatoms or heteroatom groups each independently selected from the group consisting of N, O, S, and NH;
R₁ is selected from the group consisting of H, deuterium, NH₂, CH₃, CHF₂, CH₂F, and CF₃;
R₂ is selected from the group consisting of deuterium, F, Cl, Br, I, CN, COOH, -C(=O)-C₁₋₃ alkyl, -COO-C₁₋₃ alkyl, C₁₋₃ alkyl, and -C(=O)NH₂, wherein the -C(=O)-C₁₋₃ alkyl, -COO-C₁₋₃ alkyl, C₁₋₃ alkyl and -C(=O)NH₂ are each independently optionally substituted with 1, 2, or 3 Rₐ;
R₃ is selected from the group consisting of H, F, Cl, Br, I, NO₂, CN, C₁₋₃ alkyl, C₁₋₃ alkoxy, and C₁₋₃ alkylamino, wherein the C₁₋₃ alkyl, C₁₋₃ alkoxy and C₁₋₃ alkylamino are each independently optionally substituted with 1, 2, or 3 R_{b};
R₄, R₅, and R₆ are each independently selected from the group consisting of H, deuterium, F, Cl, Br, I, NH₂, NO₂, CN, OH, C₁₋₃ alkyl, C₁₋₃ alkoxy, and -NH-O-C₁₋₃ alkyl, wherein the NH₂, C₁₋₃ alkyl, C₁₋₃ alkoxy and -NH-O-C₁₋₃ alkyl are each independently optionally substituted with 1, 2, or 3 R_{c};
each Rₐ is independently selected from the group consisting of deuterium, F, Cl, Br, I, OH, and NH₂;
each R_{b} is independently selected from the group consisting of deuterium, F, Cl, Br, I, and OH;
each R_{c} is independently selected from the group consisting of deuterium, F, Cl, Br, I, NH₂, and C₁₋₃ alkyl.

4. The compound, the stereoisomer thereof, or the pharmaceutically acceptable salt thereof according to any one of claims 1-3, wherein ring A is selected from the group consisting of C₆₋₁₀ aryl and 5- to 10-membered heteroaryl;
or ring A is selected from the group consisting of C₆₋₁₀ aryl and 5- to 6-membered heteroaryl;
or ring A is selected from the group consisting of phenyl and 5- to 6-membered heteroaryl;
or ring A is selected from the group consisting of aryl and 5- to 6-membered heteroaryl, wherein the 5- to 6-membered heteroaryl comprises 1, 2, or 3 heteroatoms or heteroatom groups each independently selected from the group consisting of N, O, S, and NH;
or ring A is selected from the group consisting of phenyl, pyrazolyl, and pyridinyl;
or ring A is selected from pyridinyl.

5. The compound, the stereoisomer thereof, or the pharmaceutically acceptable salt thereof according to any one of claims 1-4, wherein Rₐ, R_{b}, and R_{c} are each independently selected from the group consisting of halogen, OH, NH₂, and C₁₋₃ alkyl;
or Rₐ, R_{b}, and R_{c} are each independently selected from the group consisting of halogen, OH, and C₁₋₃ alkyl;
or Rₐ, R_{b}, and R_{c} are each independently selected from the group consisting of fluorine, OH, and C₁₋₃ alkyl;
or each Rₐ is independently selected from OH;
or each R_{c} is independently selected from the group consisting of F and CH₃.

6. The compound, the stereoisomer thereof, or the pharmaceutically acceptable salt thereof according to any one of claims 1-5, wherein R₁ is selected from the group consisting of H, deuterium, NH₂, and C₁₋₃ alkyl optionally substituted with one or more halogens, CN, or OH;
or R₁ is selected from the group consisting of H, deuterium, NH₂, and C₁₋₃ alkyl optionally substituted with one or more halogens;
or R₁ is selected from the group consisting of H, deuterium, NH₂, and C₁₋₃ alkyl optionally substituted with one or more fluorine;
or R₁ is selected from the group consisting of H, deuterium, NH₂, CH₃, CHF₂, CH₂F, and CF₃;
or R₁ is selected from the group consisting of H, CH₃, and CHF₂; or R₁ is selected from the group consisting of CH₃ and CHF₂.

7. The compound, the stereoisomer thereof, or the pharmaceutically acceptable salt thereof according to any one of claims 1-6, wherein R₂ is selected from the group consisting of deuterium, F, Cl, Br, I, CN, COOH, -C(=O)-C₁₋₃ alkyl, -COO-C₁₋₃ alkyl, C₁₋₃ alkyl, and -C(=O)NH₂, wherein the -C(=O)-C₁₋₃ alkyl, -COO-C₁₋₃ alkyl, C₁₋₃ alkyl and -C(=O)NH₂ are each independently optionally substituted with 1, 2, or 3 Rₐ;
or R₂ is selected from the group consisting of deuterium, F, Cl, Br, I, CH₃, and -C(=O)NH₂, wherein the CH₃ and -C(=O)NH₂ are each independently optionally substituted with 1, 2, or 3 Rₐ; or R₂ is selected from the group consisting of Cl, -CH₂OH, -CH₃, and -C(=O)NH₂;
or R₂ is selected from the group consisting of Cl, -CH₂OH, and -C(=O)NH₂.

8. The compound, the stereoisomer thereof, or the pharmaceutically acceptable salt thereof according to any one of claims 1-7, wherein R₃ is selected from the group consisting of H, F, Cl, Br, I, NO₂, CN, C₁₋₃ alkyl, C₁₋₃ alkoxy, and C₁₋₃ alkylamino, wherein the C₁₋₃ alkyl, C₁₋₃ alkoxy and C₁₋₃ alkylamino are each independently optionally substituted with 1, 2, or 3 R_{b};
or R₃ is selected from the group consisting of H, F, Cl, and CH₃; or R₃ is selected from H.

9. The compound, the stereoisomer thereof, or the pharmaceutically acceptable salt thereof according to any one of claims 1-8, wherein R₄, R₅, and R₆ are each independently selected from the group consisting of H, deuterium, F, Cl, Br, I, NH₂, NO₂, CN, OH, C₁₋₃ alkyl, C₁₋₃ alkoxy, and -NH-O-C₁₋₃ alkyl, wherein the NH₂, C₁₋₃ alkyl, C₁₋₃ alkoxy and -NH-O-C₁₋₃ alkyl are each independently optionally substituted with 1, 2, or 3 R_{c};
or R₄, R₅, and R₆ are each independently selected from the group consisting of H, deuterium, F, Cl, Br, I, NH₂, NO₂, CN, OH, =O, CH₃, -OCH₃, and -NH-O-CH₃, wherein the NH₂, CH₃, -OCH₃ and -NH-O-CH₃ are each independently optionally substituted with 1, 2, or 3 R_{c};
or R₄, R₅, and R₆ are each independently selected from the group consisting of F, Cl, NH₂, -NH-O-CH₃, -NH-CH₃, CH₃, CF₃, and CHF₂.

10. The compound, the stereoisomer thereof, or the pharmaceutically acceptable salt thereof according to any one of claims 1-9, wherein the structural unit 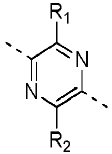 is selected from the group consisting of 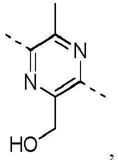 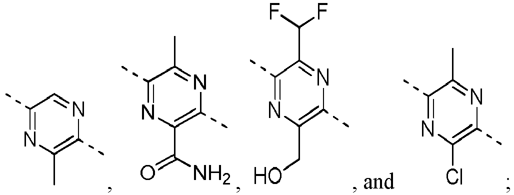 and/or the structural unit 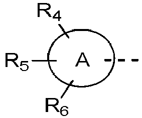 is selected from the group consisting of

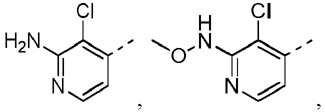

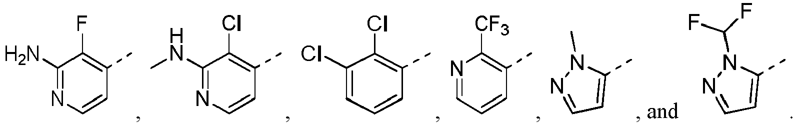

11. The compound, the stereoisomer thereof, or the pharmaceutically acceptable salt thereof according to any one of claims 1-10, wherein the compound is selected from the group consisting of 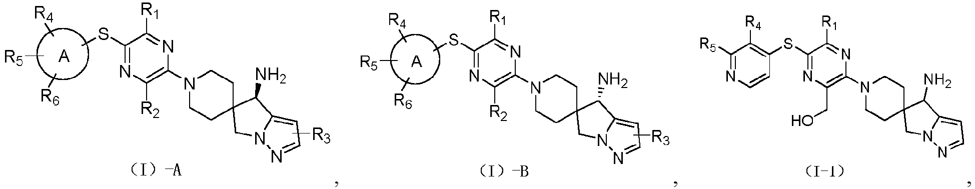 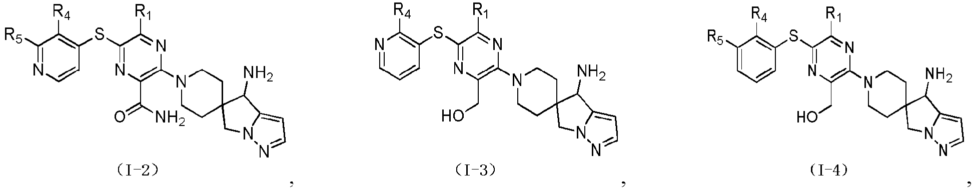 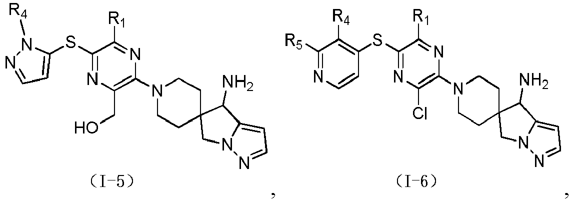 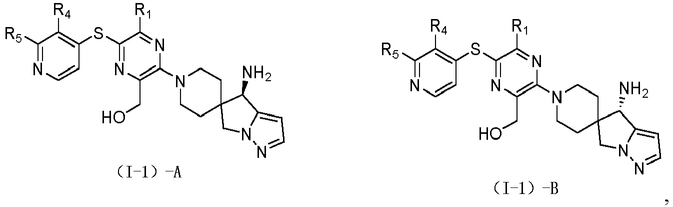 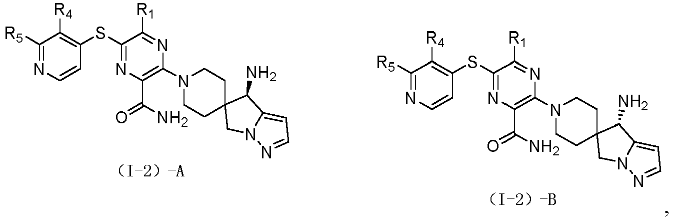 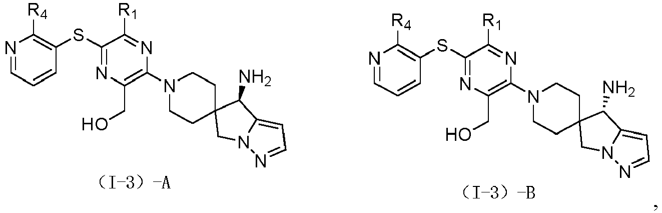 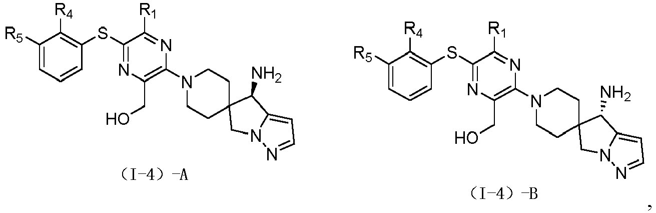 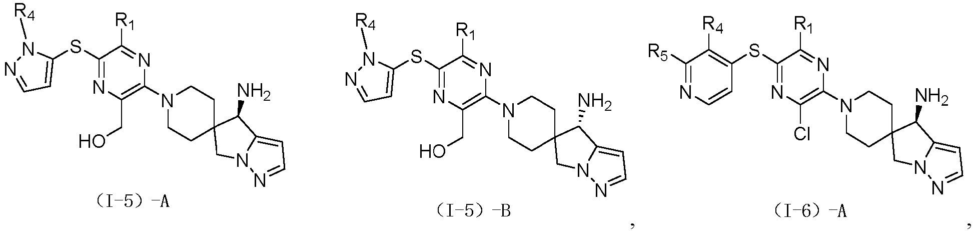 and

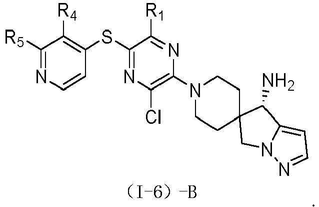

12. A compound, a stereoisomer thereof, or a pharmaceutically acceptable salt thereof, wherein the compound is selected from the group consisting of

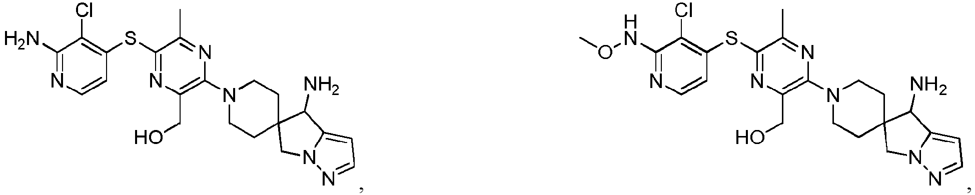

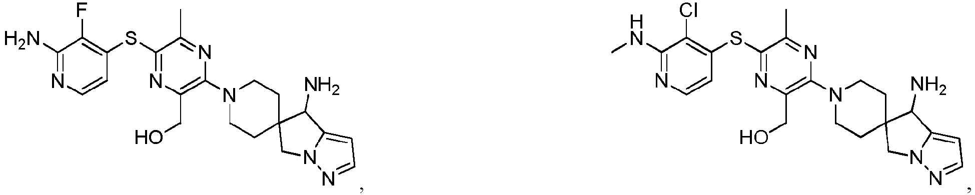

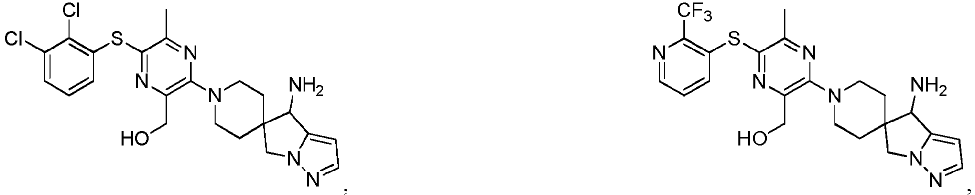

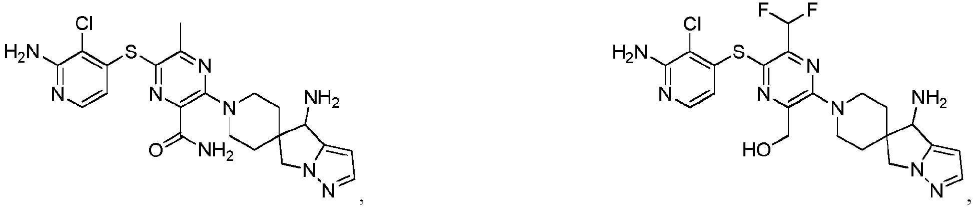

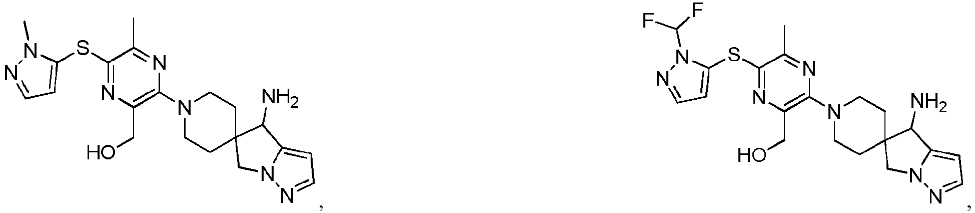

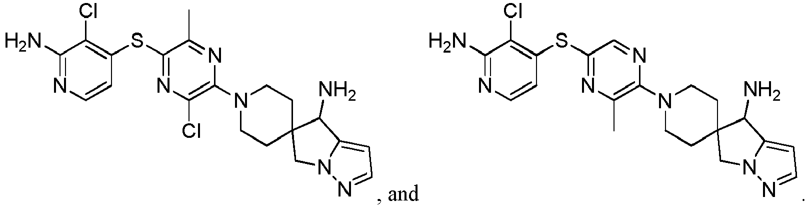

13. A compound, a stereoisomer thereof, or a pharmaceutically acceptable salt thereof, wherein the compound is selected from the group consisting of

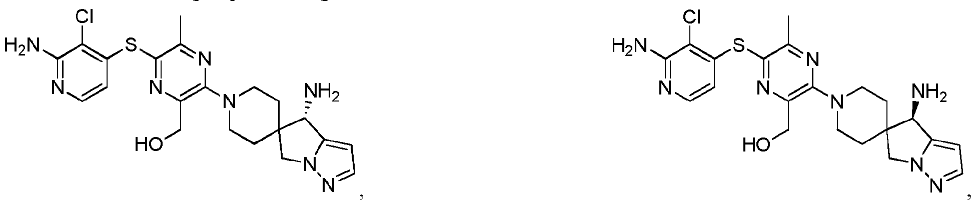

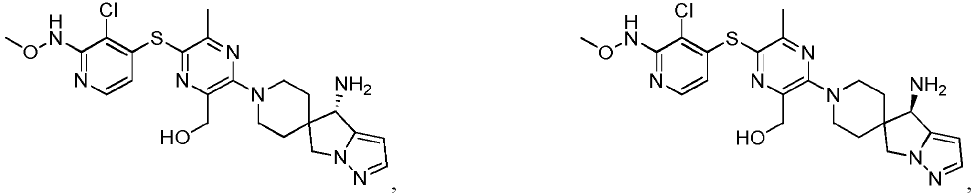

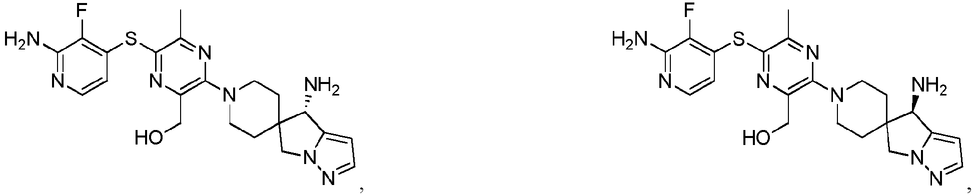

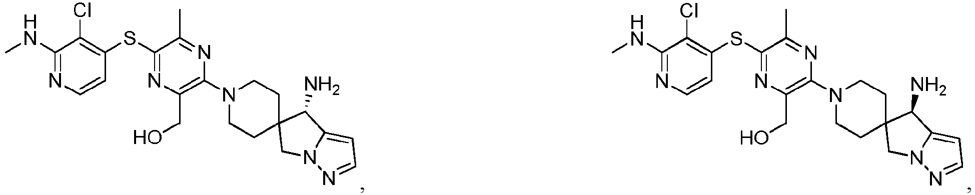

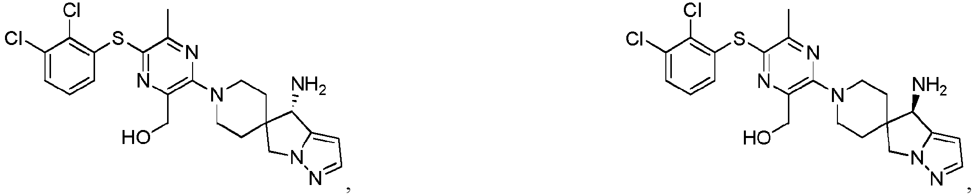

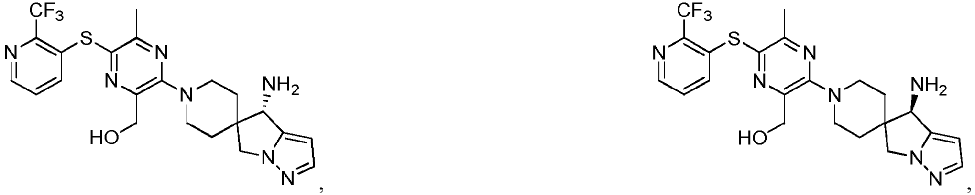

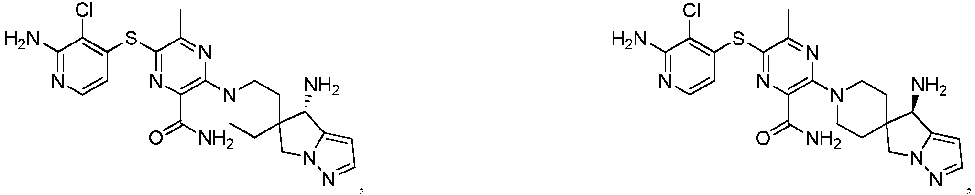

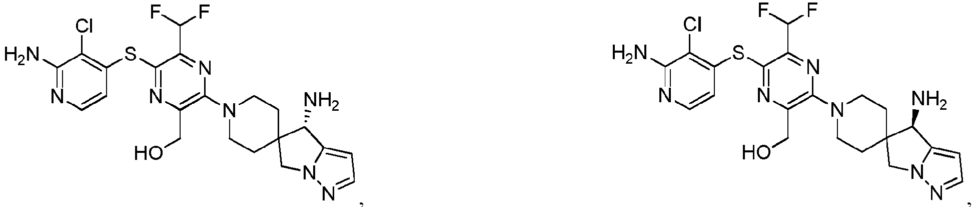

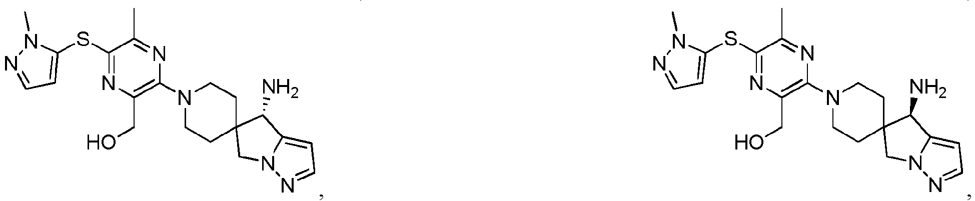

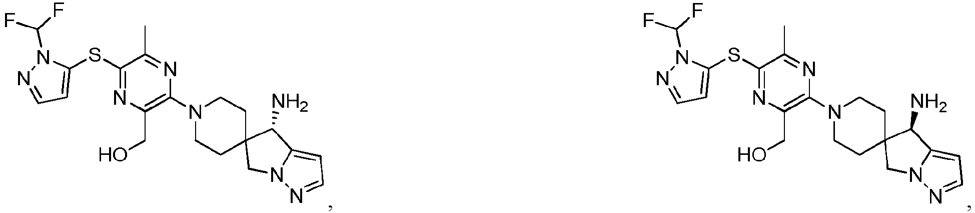

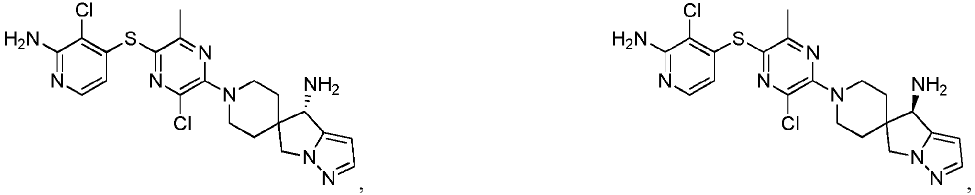

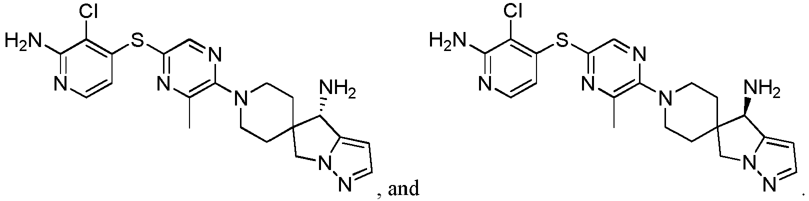

14. A pharmaceutical composition, comprising the compound, the stereoisomer thereof, or the pharmaceutically acceptable salt thereof according to any one of claims 1-13.

15. Use of the compound, the stereoisomer thereof, or the pharmaceutically acceptable salt thereof according to any one of claims 1-13 or the pharmaceutical composition according to claim 14 for preparing a medicament for preventing or treating an SHP2 protein-related disease, wherein optionally, the SHP2 protein-related disease is selected from cancer; optionally, the SHP2 protein-related disease is selected from the group consisting of lung cancer and pancreatic cancer.
